# FASCICULE DE BREVET EUROPEEN

(11) **EP 1 416 908 B1**
(45) Date de publication et mention de la délivrance du brevet: **29.10.2008**
(21) Numéro de dépôt: 02747518.5
(22) Date de dépôt: 07.06.2002
(51) Int. Cl.: A61Q 5/10, A61K 8/41, A61K 8/49

(54) **COMPOSITION DE TEINTURE DES FIBRES KERATINIQUES HUMAINES AVEC DES COLORANTS D'OXYDATION ET DES COMPOSES DICATIONIQUES**
ZUSAMMENSETZUNG ZUR FÄRBUNG VON MENSCHLICHEN KERATINISCHEN FASERN ENTHALTEND OXIDATIONSFARBSTOFFE UND DIKATIONISCHE VERBINDUNGEN
DYEING COMPOSITION FOR HUMAN KERATINOUS FIBRES WITH OXIDATION DYES AND DICATIONIC COMPOUNDS

(30) Priorité: 12.06.2001 FR 0107680
(43) Date de publication de la demande: 12.05.2004
(73) Titulaire: L'ORÉAL, 75008 Paris (FR)
(72) Inventeur: PLOS, Grégory, F-75011 Paris (FR); SAMAIN, Henri, F-91570 Bievres (FR)
(74) Mandataire: Wattremez, Catherine
(86) Numéro de dépôt international: PCT/FR2002/001964
(87) Numéro de publication internationale: WO 2002/100366

(56) Documents cités:
- EP-A- 0 318 294
- EP-A- 0 696 619
- EP-A- 1 022 016
- EP-A- 1 133 975
- DE-A- 4 128 490
- DE-A- 19 802 940
- FR-A- 2 779 054
- US-A- 3 578 386
- US-A- 5 708 151

## Description

L'invention concerne une composition de teinture pour fibres kératiniques humaines et plus particulièrement les cheveux, comprenant, dans un milieu approprié pour la teinture, au moins un colorant d'oxydation, et en outre au moins un composé dicationique.

Il est connu de teindre les fibres kératiniques et en particulier les cheveux humains, avec des compositions de teinture contenant des précurseurs de colorants d'oxydation, généralement appelés "bases d'oxydation", en particulier des ortho- ou paraphénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration, ou "coupleurs", qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les "bases d'oxydation" et d'autre part par les "coupleurs", permet l'obtention d'une palette très riche en coloris.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Pour enrichir de reflets les nuances obtenues avec lesdits colorants d'oxydation, on leur ajoute généralement des colorants directs choisis le plus souvent parmi des colorants directs nitrés benzéniques, des colorants directs azoïques, des colorants directs quinoniques et en particulier anthraquinoniques, des colorants directs aziniques, triarylméthaniques, indoaminiques ou des colorants directs naturels.

Les colorants d'oxydation ont cependant l'inconvénient, lorsqu'ils sont incorporés dans des compositions tinctoriales, de conduire à des nuances qui ne sont pas très chromatiques.

La chromaticité (ou luminosité) est définie par la valeur **c**^{*} dans le système de notation colorimétrique L^{*}, a^{*}, b^{*}, de la Commission Internationale de l'Eclairage (C.I.E.). Cette valeur est égale à la racine carrée de la somme a² + b² (+a est rouge, -a est vert, +b est jaune, -b est bleu). La nuance est d'autant plus lumineuse que la valeur de c* est grande.

Dans ce système de notation, L^{*} définit la puissance de la nuance. La nuance est d'autant plus puissante que la valeur de L^{*} est faible (0=noir, 100=blanc).

On a déjà décrit et proposé certains colorants dicationiques pour colorer du papier; des azo- ou di-azoimidazoles cationiques ont ainsi été décrits dans les brevets américains N°-5 708 151 et 5 674 299.

Les colorants cationiques décrits dans la présente invention n'ont jamais été proposés pour être utilisés en cosmétique.

Voici maintenant que la demanderesse découvre qu'il est possible d'obtenir de nouvelles teintures capables de conduire à des colorations très chromatiques, et aussi très en reflets, en associant à un colorant d'oxydation conventionnel, au moins un composé dicationique convenablement sélectionné.

Les colorations obtenues sont en outre puissantes, peu sélectives, et résistent bien aux diverses agressions que peuvent subir les cheveux.

Ces découvertes sont à la base de la présente invention.

La présente invention a ainsi pour premier objet, une composition de teinture pour fibres kératiniques humaines et plus particulièrement des cheveux, comprenant, dans un milieu approprié pour la teinture, au moins un colorant d'oxydation, caractérisée par le fait qu'elle comprend en outre au moins un composé dicationique choisi parmi ceux de formules (I), (II), ou (III), décrites ci-après.

Un autre objet de l'invention porte sur une composition prête à l'emploi pour la teinture des fibres kératiniques humaines, et en particulier des cheveux, qui contient au moins un colorant d'oxydation, au moins un composé dicationique de formules (I), (II), ou (III), et un agent oxydant.

Par "composition prête à l'emploi", on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

L'invention vise également un procédé de teinture d'oxydation des fibres kératiniques humaines et en particulier des cheveux, consistant à appliquer sur les fibres au moins une composition A contenant, dans un milieu approprié pour la teinture, au moins un colorant d'oxydation et au moins un composé dicationique de formules (I), (II), ou (III), la couleur étant révélée à pH alcalin, neutre ou acide, à l'aide d'une composition B contenant au moins un agent oxydant, qui est mélangée juste au moment de l'emploi à la composition A ou qui est appliquée séquentiellement sans rinçage intermédiaire.

L'invention a également pour objet un dispositif pour la teinture d'oxydation des fibres kératiniques humaines et en particulier des cheveux, ou "kit " de teinture, qui comprend un premier compartiment renfermant, dans un milieu approprié pour la teinture, au moins un colorant d'oxydation et au moins un composé dicationique de formules (I), (II), ou (III), et un deuxième compartiment renfermant un agent oxydant.

Ce dispositif peut être équipé d'un moyen permettant de délivrer sur les cheveux le mélange souhaité, tels que les dispositifs décrits dans le brevet FR-2 586 913 au nom de la demanderesse.

Mais d'autres caractéristiques, aspects, objets et avantages de l'invention apparaîtront encore plus clairement à la lecture de la description et des exemples qui suivent.

### Colorant direct dicationique

Les colorants directs dicationiques selon la présente invention sont choisis parmi ceux de formules (I), (II), ou (III), suivantes : 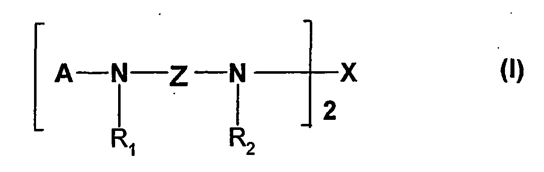 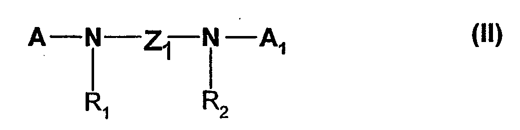 formules (I) ou (II) dans lesquelles :
- A et A1, indépendamment l'un de l'autre désignent un radical de formule (a) suivante 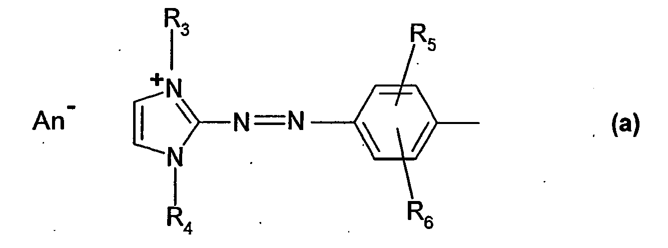
- Z désigne un radical aliphatique ou aromatique,
- Z₁ désigne un radical alkyle,
- R₁ et R₂, indépendamment l'un de l'autre, désignent un atome d'hydrogène, ou un radical (C₁-C₄)alkyl, ou (C₁-C₄)alkyl substitué par un ou plusieurs atomes d'halogène, un radical hydroxyl, carboxyl, cyano, un radical (C₁-C₄)alcoxy, un radical (C₁-C₄)alcoxy substitué par un ou plusieurs radicaux hydroxyl, ou (C₁-C₄)alcoxy, un radical amino, alkylamino, dialkylamino, aminocarbonyl, phényl, phénoxy ou phénylaminocarbonyl, dans lequel le radical phényl est non substitué ou substitué par un radical (C₁-C₄)alkyl, (C₁-C₄)alcoxy ou phénoxy,
ou encore R₁ et R₂ forment ensemble, avec les deux atomes d'azote qui les portent et le radical Z, un cycle pipérazinique,
- X est un radical de pontage choisi parmi : -CO- ; -CO-CH₂-CH₂-CO- ; -CO-CO- ; 1,4-dicarbonylphényl ; -CH₂-CH₂- ; ou une triazine de formules (b) ou (c) suivantes: 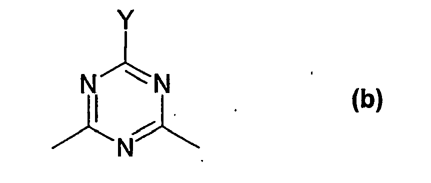
ou 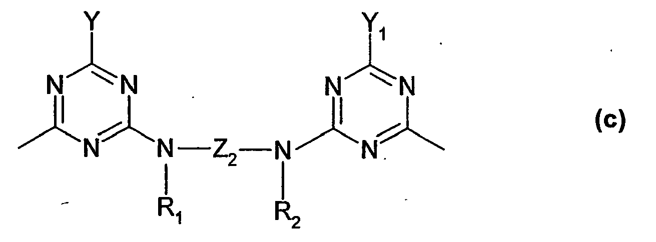 dans lesquelles :
Y et Y1, indépendamment l'un de l'autre désignent un atome d'halogène, ou un radical hydroxyl, ou amino, ou monoalkylamino, ou dialkylamino, ou 1-pipéridino, ou morpholino, ou 1-pipérazino, le radical pipérazino étant non substitué, ou substitué sur l'atome d'azote non attaché au cycle triazine par un radical (C₁-C₄)alkyl, lesdits radicaux alkyls étant non substitués ou substitués par hydroxyl, amino, mono-(C₁-C₄)alkylamino ou di-(C₁-C₄)alkylamino,

- Z₂ désigne un radical (C₂-C₈)alkylène ou forme, avec les deux atomes d'azote adjacents et les radicaux R₁ et R₂, un cycle pipérazinique,
- dans le radical de formule (a),
- R₃ et R₄, indépendamment l'un de l'autre, désignent un atome d'hydrogène, ou un radical (C₁-C₄)alkyl, ou (C₁-C₄)alkyl substitué par un ou plusieurs atomes d'halogène, un radical hydroxyl, carboxyl, cyano, un radical (C₁-C₄)alcoxy, un radical (C₁-C₄)alcoxy substitué par un radical hydroxyl ou (C₁-C₄)alcoxy, un radical amino, alkylamino, dialkylamino, aminocarbonyl, phényl, phénoxy ou phénylaminocarbonyl, dans lequel le radical phényl est non substitué ou substitué par un radical (C₁-C₄)alkyl, (C₁-C₄)alcoxy ou phénoxy,
- R₅ et R₆, indépendamment l'un de l'autre, désignent un atome d'hydrogène, un radical (C₁-C₄)alkyl ou (C₁-C₄)alcoxy éventuellement substitués par un radical hydroxyl, carboxyl, halogène, cyano, (C₁-C₄)alcoxy éventuellement substitué par un radical hydroxyl ou (C₁-C₄)alcoxy, un radical amino, alkylamino, dialkylamino, aminocarbonyl, phényl, phénoxy ou phénylaminocarbonyl, dans lequel le radical phényl est non substitué ou substitué par un radical (C₁-C₄)alkyl, (C₁-C₄)alcoxy ou phénoxy,
- An⁻ désigne un anion.

De préférence, selon l'invention, dans la formule (I),
- R₁ et R₂, indépendamment l'un de l'autre, désignent hydrogène, (C₁-C₄)alkyl substitué par hydroxyl ou (C₁-C₄)alcoxy et plus particulièrement encore désignent hydrogène ou méthyle,
- Z désigne un radical alkyle en C₂-C₈ linéaire ou ramifié ou cyclique, éventuellement substitué par un hydroxy, alcoxy, halogène, la chaîne dudit radical étant éventuellement interrompue par un groupe -O- ou -NR₁-; un radical 1,4-phényl, un radical 1,4-naphtyl éventuellement substitué par un alkyle, alcoxy, halogène ; Z pouvant formé avec R₁, R₂ et les 2 atomes d'azote, un cycle pipérazine,
- Z désigne préférentiellement un radical phényl non substitué, un radical phényl ou naphtyl substitué par 1 ou 2 radicaux méthyl ou méthoxy, un radical pipérazine par liaison avec R₁, R₂ et les 2 atomes d'azote, un radical (C₂-C₄)alkylène non subsitué ou substitué par un ou 2 hydroxyl,
- X désigne un groupement de formule (b).

De préférence, selon l'invention, dans la formule (II),
- Z1 désigne un radical alkyle en C₂-C₈ linéaire ou ramifié ou cyclique, éventuellement substitué par un hydroxy, alcoxy, halogène, la chaîne dudit radical étant éventuellement interrompue par un groupe -O- ou -NR₁- ; un cycle pipérazine formé avec R₁, R₂ et les deux atomes d'azote,
- Z₁ désigne préférentiellement un radical (C₂-C₆)alkylène non substitué ou substitué par un ou plusieurs hydroxyl, un cycle pipérazine formé avec R₁, R₂ et les deux atomes d'azote et plus particulièrement encore un radical (C₂-C₄)alkylène non substitué,
- R₃ et R₄ désignent méthyl ou éthyl, et R₅ et R₆ désignent hydrogène, méthyl, ou méthoxy. 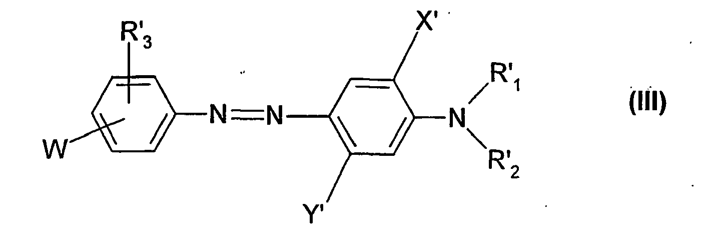 formule (III) dans laquelle,
- le nombre de charges cationiques est de deux,
- X' et Y', indépendamment l'un de l'autre, désignent hydrogène, halogène, (C₁-C₄)alkyl, (C₁-C₄)alcoxy, (C₁-C₄)alkylcarbonylamino, arylcarbonylamino, uréido, ou aryluréido,
- R'₁ désigne hydrogène, un radical alkyl ou aryl substitués, un radical alkyl ou aryl non substitués, ou la même désignation que R'₂
- R'₂ est un radical de formule (d) suivante: 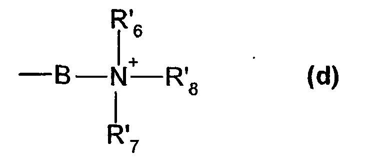
dans laquelle :
- B désigne un radical alkylène linéaire ou ramifié,
- R'₆ désigne hydrogène ou alkyl substitué ou non substitué,
- R'₇ et R'₈, indépendamment l'un de l'autre désignent alkyl substitué ou non substitué,
- R'₆ et R'₇, ensemble avec l'azote, forment un cycle à 5, 6, ou 7 chaînons, substitué ou non substitué, pouvant contenir d'autres hétéroatomes, ou bien R'₆ et R'₇ et R'₈ forment ensemble un cycle pyridinium,
- R'₃ désigne hydrogène, halogène, (C₁-C₄)alkyl, (C₁-C₄)alcoxy,
- W est un radical de formule (e) suivante : 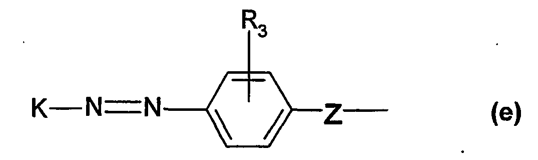 dans laquelle:

- K est un radical de couplage,
- Z désigne un radical de pontage choisi parmi les radicaux de formules :

   -NR'₉-CO-;

   -CO-NR'₉-NR'₉-CO-;

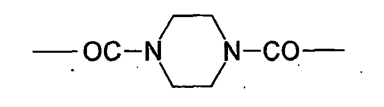

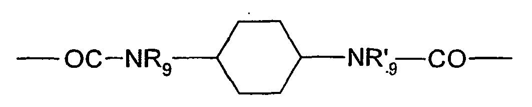

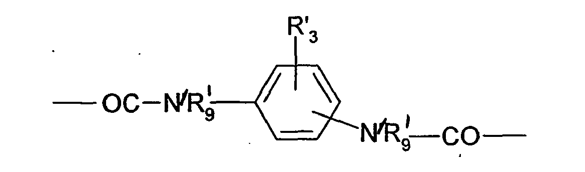

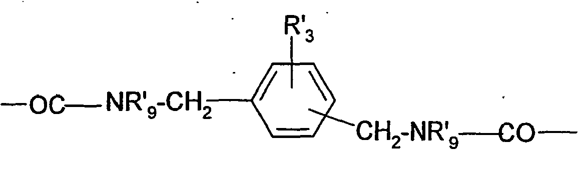

   ou bien 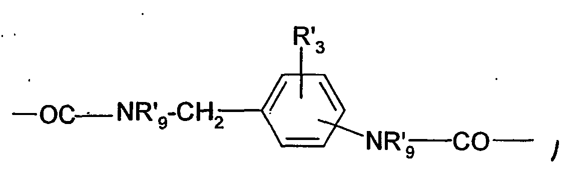 et dans lesquels R'g désigne, hydrogène, (C₂-C₄)alkylène non substitué ou substitué, le radical alkylène étant linéaire ou ramifié et pouvant être interrompu par un ou plusieurs groupements choisis parmi : -NR'9-, -O-, -S-.

De préférence, selon l'invention, dans la formule (III),
B désigne éthylène, n-propylène, isopropylène ou n-butylène,
K désigne un composé de couplage choisi parmi ceux de formule (f), (g) ou (h) suivantes 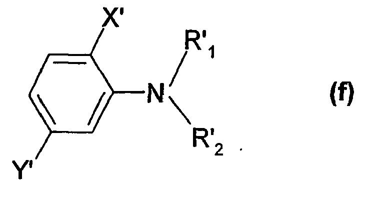 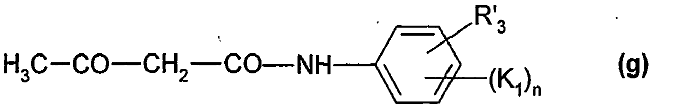 ou 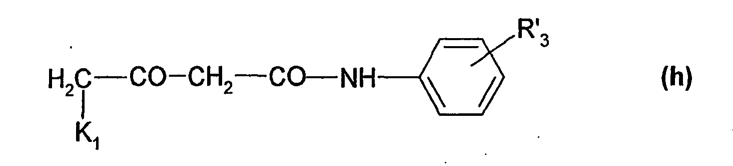 dans lesquelles,
- X', Y' et R'₁ et R'₂, ont la même désignation que dans la formule (III),
- n est égal à 1 ou 2,
- K₁ désigne le radical de formule:

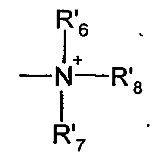

Selon la présente invention, parmi les composés de formule (I), on peut citer plus particulièrement le composé commercialisé par la société CIBA sous la dénomination PERGASOL VIOLET F-R de formule suivante :

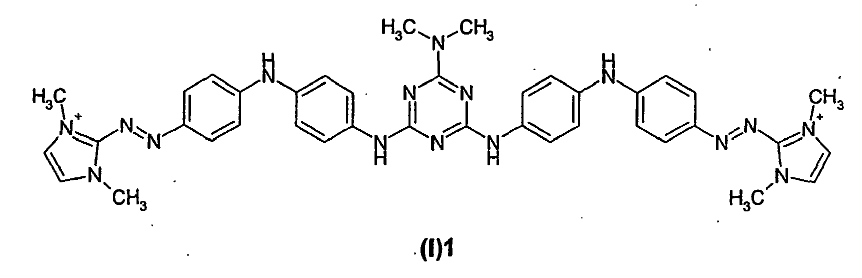

On peut également citer les composés de formule (I) ou de formule (II) répondant aux formules (I)2 à (I)7 suivantes :

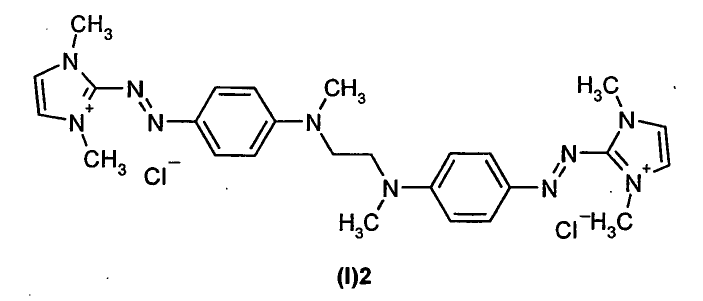

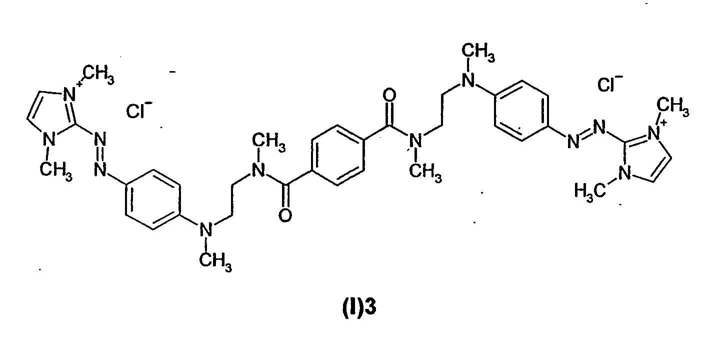

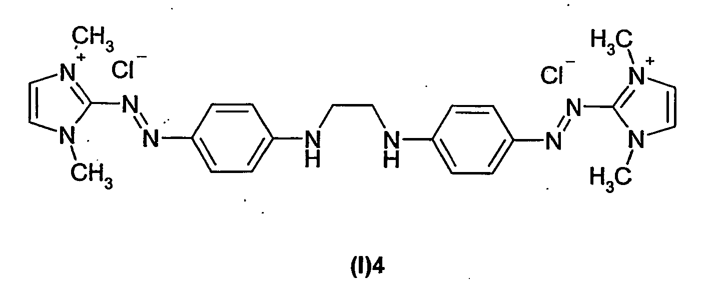

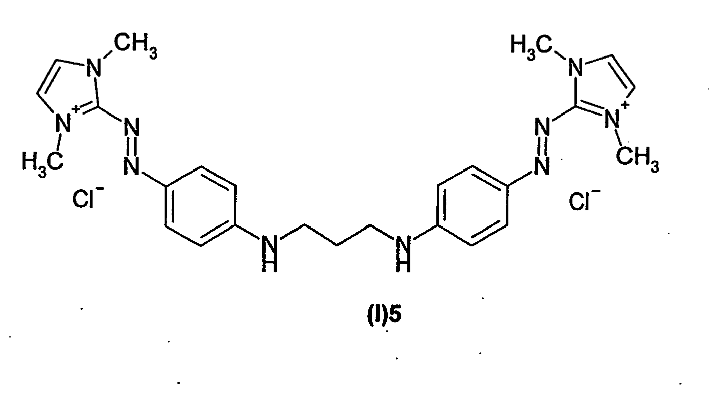

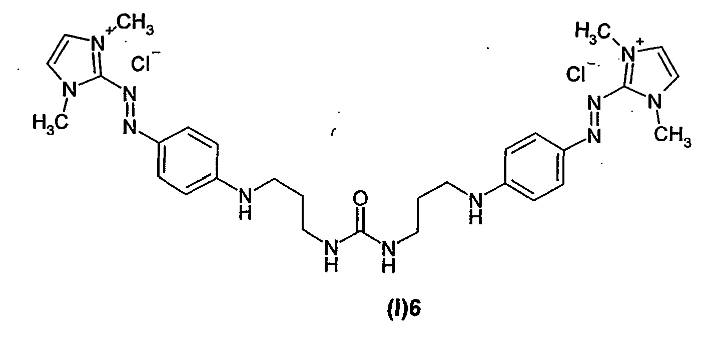

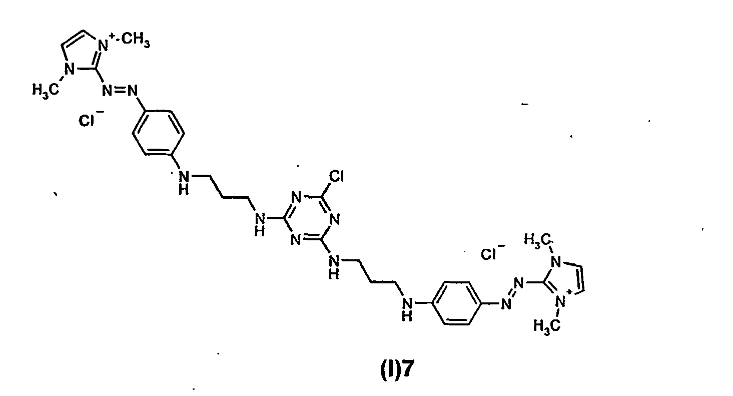

Selon la présente invention, parmi les composés de formule (III), on peut citer plus particulièrement le composé commercialisé par la société CIBA sous la dénomination PERGASOL ORANGE F-3G de formule suivante :

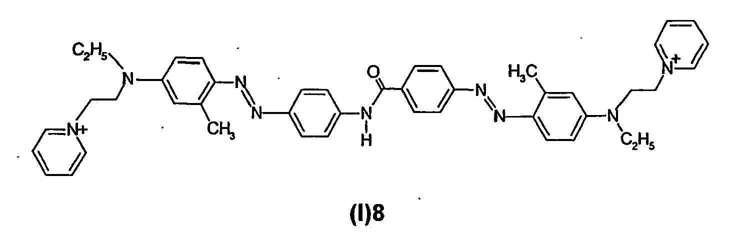

Les colorants de formule (I), (II), ou (III), selon la présente invention sont connus en tant que tels, et décrits et préparés dans les brevets US- 5 674 299 ou US- 5 708 151, dont le contenu fait partie intégrante de la présente invention.

Ils sont généralement présents dans la composition de teinture dans des proportions comprises entre environ 0,01 et 40%, de préférence entre environ 0,1 et 20% en poids, par rapport au poids total de la composition.

### Colorants d'oxydation

Les colorants d'oxydation utilisables selon l'invention sont choisis parmi les bases d'oxydation et/ou les coupleurs.

De préférence les compositions selon l'invention contiennent au moins une base d'oxydation.

Les bases d'oxydation utilisables dans le cadre de la présente invention sont choisies parmi celles classiquement connues en teinture d'oxydation, et parmi lesquelles on peut notamment citer .les ortho- et para-phénylènediamines, les bases doubles, les ortho- et para- aminophénols, les bases hétérocycliques suivantes ainsi que leurs sels d'addition avec un acide.

On peut notamment citer :
- (A) les paraphénylènediamines de formule (V) suivante et leurs sels d'addition avec un acide : 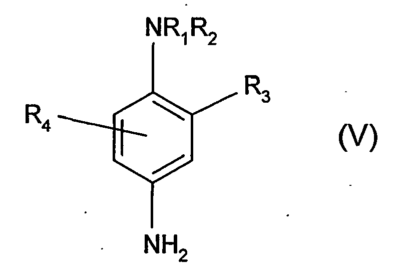 dans laquelle :
   R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄ représente un atome d'hydrogène, d'halogène ou un radical alkyle en C₁-C₄.

   Parmi les groupements azotés de la formule (V) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les paraphénylènediamines de formule (V) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.
   Parmi les paraphénylènediamines de formule (V) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- (B) Selon l'invention, on entend par bases doubles, les composés comportant au moins deux noyaux aromatiques sur lesquels sont portés des groupements amino et/ou hydroxyle.
   Parmi les bases doubles utilisables à titre de bases d'oxydation dans les compositions tinctoriales conformes à l'invention, on peut notamment citer les composés répondant à la formule (VI) suivante, et leurs sels d'addition avec un acide : 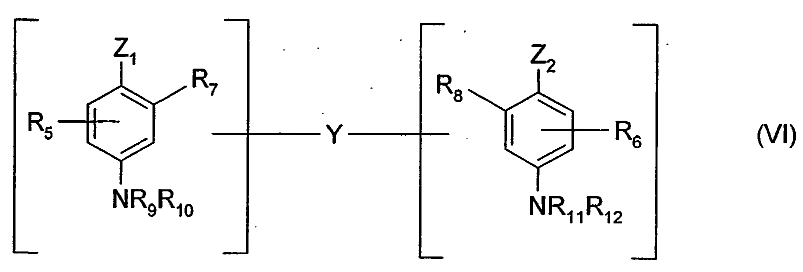 dans laquelle :
   - Z₁ et Z₂, identiques ou différents, représentent un radical hydroxyle ou -NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
   - R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   - R₇, R₈, R₉, R₁₀, R₁₁ et R₁₂, identiques ou différents, représentent un atome d'hydrogène, un bras de liaison Y ou un radical alkyle en C₁-C₄ ;
      étant entendu que les composés de formule (VI) ne comportent qu'un seul bras de liaison Y par molécule.

   Parmi les groupements azotés de la formule (VI) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (VI) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.
   Parmi ces bases doubles de formule (VI), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- (C) les para-aminophénols répondant à la formule (VII) suivante, et leurs sels d'addition avec un acide : 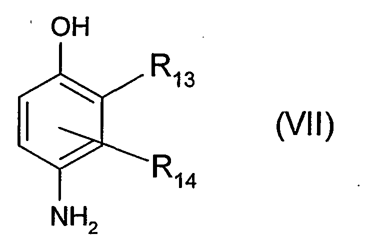 dans laquelle :
   **R₁₃** représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   **R₁₄** représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

   Parmi les para-aminophénols de formule (VII) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- (D) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- (E) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, là 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

Les coupleurs utilisables dans le procédé de teinture selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.
Lorsqu'ils sont présents, ces coupleurs représentent de préférence de 0,0001 à 10% en poids environ du poids total de la composition, et encore plus préférentiellement de 0,005 à 5% en poids environ.
D'une manière générale, les sels d'addition avec un acide des bases d'oxydation et coupleurs sont notamment choisis parmi les chlorhydrates, les bromhydrates, les sulfates et les tartrates, les lactates et les acétates.

Le milieu de la composition approprié pour la teinture, est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations allant d'environ 0,5 à 20% et, de préférence, d'environ 2 à 10% en poids par rapport au poids total de la composition.

La composition de teinture peut encore contenir une quantité efficace d'autres agents, par ailleurs antérieurement connus en coloration d'oxydation, tels que divers adjuvants usuels comme des agents tensio-actifs bien connus de l'état de la technique et de type anionique, cationique, non-ionique, amphotère, zwittérionique ou leurs mélanges, des agents épaississants, des agents anti-oxydants, des parfums, des agents dispersants, des agents de conditionnement dont notamment des polymères cationiques ou amphotères, des agents opacifiants, des agents séquestrants tel que l'EDTA et l'acide étidronique, des filtres UV, des cires, des silicones volatiles ou non, cycliques ou linéaires ou ramifiées, organomodifiées (notamment par des groupements amines) ou non, des agents conservateurs, des céramides, des pseudocéramides, des huiles végétales, minérales ou de synthèse, les vitamines ou provitamines comme le panthénol, des polymères associatifs non-ioniques, anioniques, amphotères ou cationiques.

### Polymères associatifs

Les polymères associatifs sont des polymères hydrosolubles capables, dans un milieu aqueux, de s'associer réversiblement entre eux ou avec d'autres molécules.
Leur structure chimique comprend des zones hydrophiles, et des zones hydrophobes caractérisées par au moins une chaîne grasse.

### Polymères associatifs de type anionique :

On peut citer parmi eux :
- **(I)** ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (I) suivante :

   CH₂ = C R' CH₂ O Bₙ R (I)
dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryl (C₁₈).
Des polymères associatifs anioniques de ce type sont décrits et préparés, selon un procédé de polymérisation en émulsion, dans le brevet EP-0 216 479.
Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (I), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthytène-bis-acrylamide.
Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).
- **(II)** ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (II) suivante : 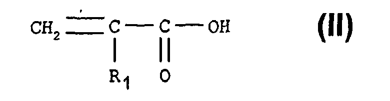
dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (III) suivante 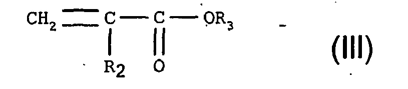 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.
Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
Des polymères anioniques de ce type sont par exemple décrits et préparés, selon les brevets US-3 915 921 et 4 509 949.
Parmi ce type de polymères associatifs anioniques, on utilisera plus particulièrement des polymères formés à partir d'un mélange de monomères comprenant :
(i) essentiellement de l'acide acrylique,
(ii) un ester de formule (III) décrite ci-dessus et dans laquelle R₂ désigne H ou CH₃, R₃ désignant un radical alkyle ayant de 12 à 22 atomes de carbone,
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
Parmi ce type de polymères associatifs anioniques, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkylés en C₁₀-C₃₀ (motif hydrophobe), et 0,1 à 0,6% en poids de monomère polymérisable, réticulant tel que ceux décrits précedemment.
Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX.
- **(III)** les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608 par la société NEWPHASE TECHNOLOGIES.
- **(IV)** les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monoéthylénique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
      tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (40OE) en dispersion aqueuse à 25%.
- **(V)** les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C1C4.
A titre d'exemple de ce type de composé on peut citer l'ACULYN 22 vendu par la société ROHM et HAAS, qui est un terpolymère acide méthacrylique/acrylate d'éthyle/méthacrylate de stéaryle oxyalkyléné.

### Polymères associatifs de type cationique

Selon la présente invention, ils sont choisis de préférence parmi les dérivés de cellulose quaternisée et les polyacrylates à groupements latéraux aminés non cycliques.

Les dérivés de cellulose quaternisée sont, en particulier,
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quaternisées à chaînes grasses en C₈-C₃₀, les produits QUATRISOFT LM 200, QUATRISOFT LM-X 529-18-A, QUATRISOFT LM-X 529-18B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL QS (alkyle en C18) commercialisés par la société CRODA.

### Polymères associatifs amphotères

Ils sont choisis de préférence parmi ceux comportant au moins un motif cationique non cyclique. Plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.

Les polymères associatifs amphotères préférés selon l'invention comprennent, ou sont préparés en copolymérisant :
1) au moins un monomère de formule (la) ou (Ib): 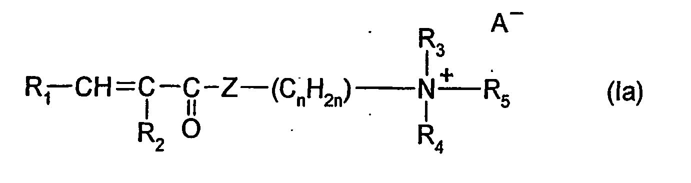 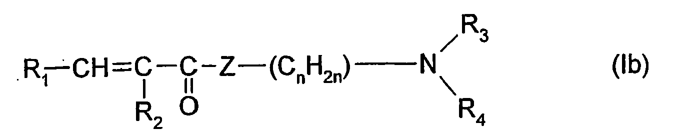 dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A- est un anion issu d'un acide organique ou minéral, tel qu'un anion méthosulfate ou un halogénure tel que chlorure ou bromure;
2) au moins un monomère de formule (II)

   R₆-CH= CR₇-COOH (II)

   dans laquelle, R₆ et R₇, identiques ou différents, représentent un atome d'hydrogène ou un radical méthyle;
   et
3) au moins un monomère de formule (III) :

   R₆-CH= CR₇- COXR₈ (III)
dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
l'un au moins des monomères de formule (Ia), (Ib) ou (III) comportant au moins une chaîne grasse.

Les monomères de formule (Ia) et (Ib) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,
   ces monomères étant éventuellement quatemisés, par exemple par un halogénure d'alkyle en C₁-C₄ ou un sulfate de dialkyle en C₁-C₄.

Plus particulièrement, le monomère de formule (la) est choisi parmi le chlorure d'acrylamidopropyl triméthyl ammonium et le chlorure de méthacrylamidopropyl triméthyl ammonium.

Les monomères de formule (II) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (II) est l'acide acrylique.

Les monomères de formule (III) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères selon l'invention comprennent de préférence de 1 à 10 moles% du monomère comportant une chaîne grasse (monomère de formule (Ia), (Ib) ou (III)), et de préférence de 1,5 à 6 moles %,

Les poids moléculaires moyens en poids des polymères associatifs amphotères selon invention peuvent varier de 500 à 50.000.000 et sont de préférence compris entre 10.000 et 5 000 000.

Les polymères associatifs amphotères selon l'invention peuvent également contenir d'autre monomères tels que des monomères non ioniques et en particulier tels que les acrylates ou méthacrylates d'alkyle en C₁-C₄.

Des polymères associatifs amphotères sont par exemple décrits et préparés dans la demande de brevet W09844012.

Parmi les polymères associatifs amphotères selon l'invention, on préfère les terpolymères acide acrylique/chlorure de (méth)acrylamidopropyl triméthyl ammonium/ méthacrylate de stéaryle.

### Polymères associatifs de type non ionique

Selon l'invention, ils sont choisis de préférence parmi :
- **(1)** les celluloses modifiées par des groupements comportant au moins une chaîne grasse ; on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
   - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
- **(2)** les hydroxypropylguars modifiés par des groupements comportant au moins une chaîne grasse tel que le produit ESAFLOR HM 22 (chaîne alkyle en C₂₂) vendu par la société LAMBERTI, les produits RE210-18 (chaîne alkyle en C₁₄) et RE205-1 (chaîne alkyle en C₂₀) vendus par la société RHONE POULENC.
- **(3)** les copolymères de vinyl pyrrolidone et de monomères hydrophobes à chaîne grasse dont on peut citer à titre d'exemple:
   - les produits ANTARON V216 ou GANEX V216 (copolymère vinylpyrrolidone /hexadécène) vendu par la société I.S.P.
   - les produits ANTARON V220 ou GANEX V220 (copolymère vinylpyrrolidone /eicosène) vendu par la société I.S.P.
- **(4)** les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.
- **(5)** les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- **(6)** les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- **(7)** les polymères à squelette aminoplaste éther possédant au moins une chaîne grasse, tels que les composés PURE THIX proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.
Par extension figurent aussi parmi les polyéthers polyuréthanes non-ioniques à chaîne grasse, ceux dont les séquences hydrophiles sont liées aux séquences lipophiles par d'autres liaisons chimiques.
A titre d'exemples de polyéthers polyuréthanes non-ioniques à chaîne grasse utilisables dans l'invention, on peut aussi utiliser aussi le Rhéolate 205 à fonction urée vendu par la société RHEOX ou encore les Rhéolates 208 , 204 ou 212, ainsi que l'Acrysol RM 184.
On peut également citer le produit ELFACOS T210 à chaîne alkyle en C₁₂₋₁₄ et le produit ELFACOS T212 à chaîne alkyle en C₁₈ de chez AKZO.

Le produit DW 1206B de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate 255, le Rhéolate 278 et le Rhéolate 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate. De tels polyéther polyuréthanes sont vendus notamment par la société ROHM & HAAS sous les appellations Aculyn 46 et Aculyn 44 [l'ACULYN 46 est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44 est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

Les polymères associatifs de type non ionique, anionique, cationique ou amphotère sont utilisés de préférence en une quantité pouvant varier d'environ 0,1 à 10% en poids du poids total de la composition colorante. Plus préférentiellement, cette quantité varie d'environ 0,5 à 5% en poids, et encore plus particulièrement d'environ 1 à 3% en poids.

### Polymères cationiques.

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :
**(1)** Les homopolymères ou copolymères dérivés d'esters ou d'amides acryliques ou méthacryliques et comportant au moins un des motifs de formules (I), (II), (III) ou (IV) suivantes: 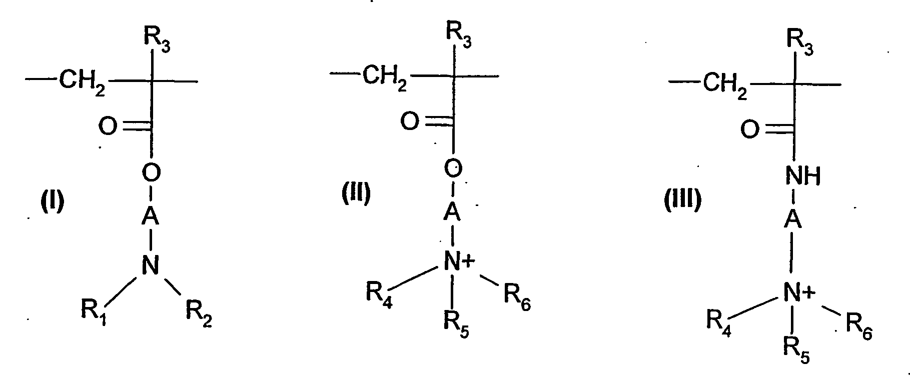 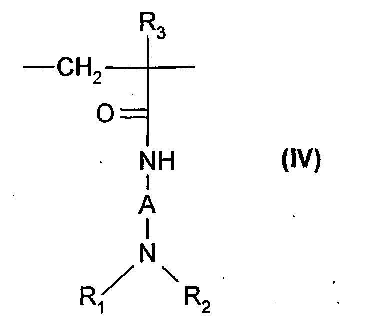 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
   - les copolymères d'acrylamide et de diméthylaminoéthyl méthacrylate quatemisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthyl-ammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthyl-ammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZ CC 10 par ISP,
   - et les copolymères vinylpyrrolidone / méthacrylamide de diméthylaminopropyle quaternisés tel que le produit vendu sous la dénomination "GAFQUAT HS 100" par la société ISP .
**(2)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quatemisation de ces polymères. De tels polymères sont notamment décrits dans les brevets français 2.162.025 et 2.280.361.
**(3)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508. **(4)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
**(5)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
**(6)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 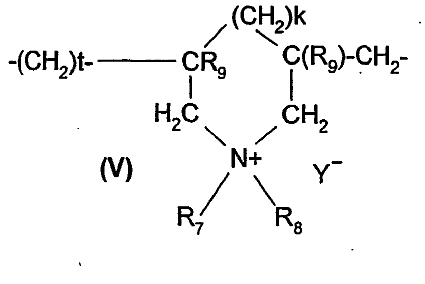 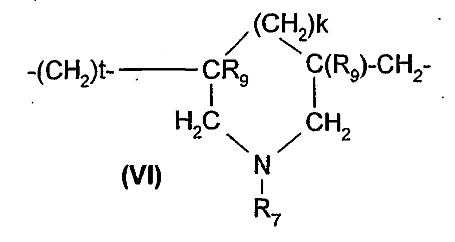 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
**(7)** Le polymère de diammonium quaternaire contenant des motifs récurrents répondant à la formule : 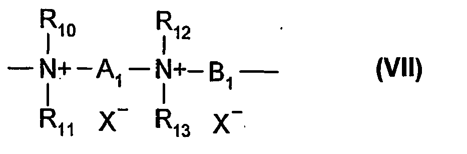 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH-
   R₁₄-D où R₁₄ est un alkylène et D un groupement ammonium quaternaire ;
      A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un
   ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - (CH₂)n-CO-D-OC-(CH₂)n- dans lequel n est compris entre 1 et 100 et de préférence entre 1 et 50, et D désigne :
   a) un reste de glycol de formule : -O-Z-O-, où Z désigne un radical hydrocarboné linéaire ou ramifié ou un groupement répondant à l'une des formules suivantes :

      -(CH₂-CH₂-O)x-CH₂-CH₂-

      -[CH₂-CH(CH₃)-O]y-CH₂-CH(CH₃)-

      où x et y désignent un nombre entier de 1 à 4, représentant un degré de polymérisation défini et unique ou un nombre quelconque de 1 à 4 représentant un degré de polymérisation moyen;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH₂-CH₂-S-S-CH₂-CH₂- ;
   d) un groupement uréylène de formule : -NH-CO-NH-.
      De préférence, X⁻ est un anion tel que le chlorure ou le bromure.
      Ces polymères ont une masse moléculaire moyenne en nombre généralement comprise entre 1000 et 100000.
      Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
      On peut utiliser plus particulièrement les polymères qui sont constitués de motifs récurrents répondant à la formule (VIII) suivante: 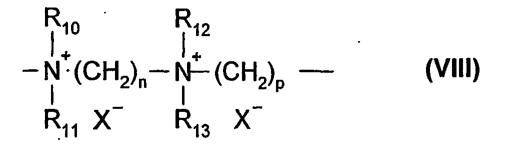 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
**(8)** Les polymères de polyammonium quaternaire constitués de motifs de formule (IX) 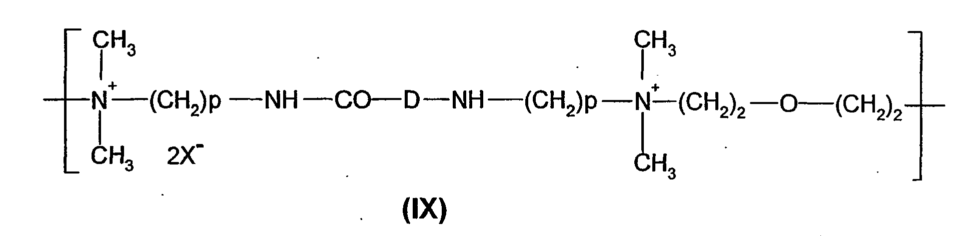 dans laquelle:
   p désigne un nombre entier variant de 1 à 6 environ,
   D peut être nul ou peut représenter un groupement -(CH₂)ᵣ -CO- dans lequel r désigne un nombre égal à 4 ou à 7, et
   X⁻ est un anion dérivé d'un acide minéral ou organique.

Les polymères cationiques comportant des motifs de formule (IX) sont notamment décrits dans la demande de brevet EP-A-122 324 et peuvent être préparés selon les procédés décrits dans les brevets U.S.A. n° 4 157 388, 4 390 689, 4 702 906, 4 719 282.

Parmi ces polymères, on préfère ceux de masse moléculaire mesurée par RMN du Carbone 13 inférieure à 100000, et dans la formule de laquelle :
p est égal à 3, et,
   a) D représente un groupement -(CH₂)₄ -CO-, X désigne un atome de chlore, la masse moléculaire mesurée par RMN du Carbone 13 ( RMN¹³C ) étant d'environ 5600 ; un polymère de ce type est proposé par la société MIRANOL sous le nom de MIRAPOL-AD1,
   b) D représente un groupement -(CH₂)₇ -CO -, X désigne un atome de chlore, la masse moléculaire mesurée par RMN du Carbone 13 ( RMN¹³C ) étant d'environ 8100 ; un polymère de ce type est proposé par la société MIRANOL sous le nom de MIRAPOL-AZ1,
   c) D désigne la valeur zéro, X désigne un atome de chlore, la masse moléculaire mesurée par RMN du Carbone 13, ( RMN¹³C ) étant d'environ 25500 ; un polymère de ce type est vendu par la société MIRANOL sous le nom MIRAPOL-A15,
   d) un " Block Copolymer " formé de motifs correspondant aux polymères décrits aux alinéas a) et c), proposé par la société MIRANOL sous les noms MIRAPOL-9, (masse moléculaire RMN¹³C, environ 7800) MIRAPOL-175, (masse moléculaire RMN¹³C, environ 8000) MIRAPOL-95, (masse moléculaire RMN¹³C, environ 12500). Plus particulièrement encore, on préfère selon l'invention le polymère à motifs de formule (IX) dans laquelle p est égal à 3, D désigne la valeur zéro, X désigne un atome de chlore, la masse moléculaire mesurée par RMN du Carbone 13,
      ( RMN¹³C ) étant d'environ 25500.
**(9)** Les polymères quaternaires de vinylpyrrolidone et de vinylimidazole tels que par exemple les produits commercialisés sous les dénominations Luviquat FC 905, FC 550 et FC 370 par la société B.A.S.F.
**(10)** Les polyamines comme le Polyquart H vendu par HENKEL, référencé sous le nom de "POLYETHYLENEGLYCOL (15) TALLOW POLYAMINE" dans le dictionnaire CTFA.
**(11)** Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quatemisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de "SALCARE^{®} SC 92" par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (6), (7) (8) et (11) et encore plus préférentiellement les polymères aux motifs récurrents de formules (W) et (U) suivantes : 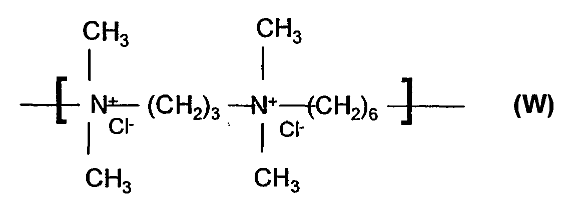 et notamment ceux dont le poids moléculaire, déterminé par chromatographie par perméation de gel, est compris entre 9500 et 9900; 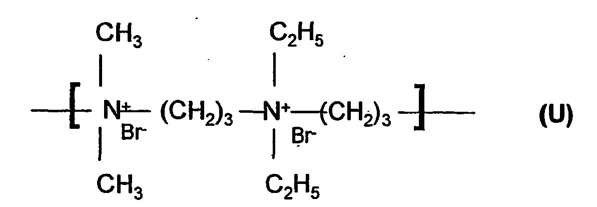 et notamment ceux dont le poids moléculaire, déterminé par chromatographie par perméation de gel, est d'environ 1200.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique substitué contenant au moins un atome basique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
      Les comonomères basiques préférés sont des méthacrylates d'aminoéthyle, de butyl aminoéthyle, de N,N'-diméthylaminoéthyle, de N-tertio-butylaminoéthyle. On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   ⁅CO-R₁₉-CO-Z⁆ **(X)**

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 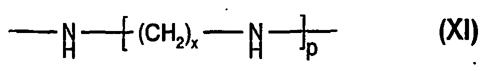 où x=2 et p=2 ou 3, ou bien x=3 et p=2 ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 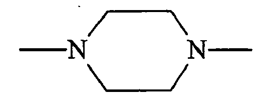
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium
   ou de potassium.
(4) Les polymères comportant des motifs zwittérioniques de formule : 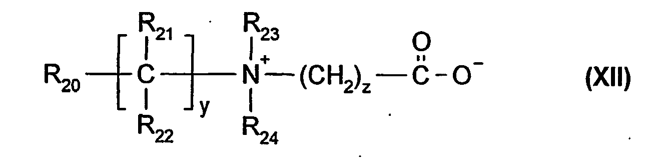 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10. Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.
(5) les polymères dérivés du chitosane décrits notamment dans les brevets français N°-2137684 ou US-3879376, comportant des motifs monomères répondant aux formules (XIII), (XIV), (XV) suivantes réunies dans leur chaîne: 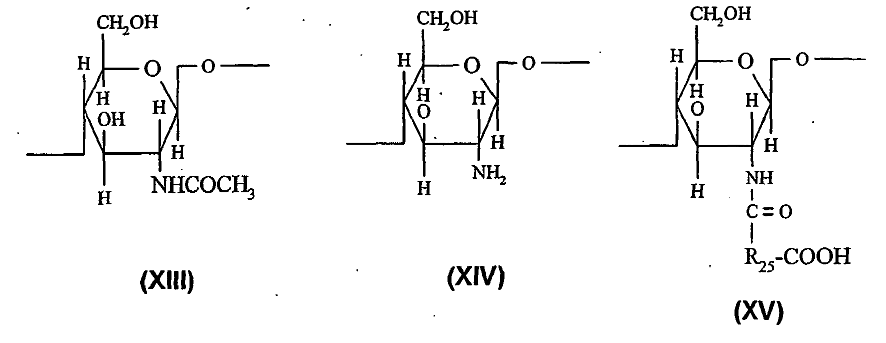 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formule : 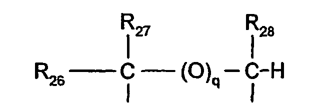 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine où un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
   Des polymères de ce type plus particulièrement préférés comportent de 0 à 20% en poids de motifs (XIII), de 40 à 50% en poids de motifs (XIV), et de 40 à 50% en poids de motifs (XV) dans lequel R₂₅ désigne le radical -CH₂-CH₂- ;
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XI) tels que ceux décrits par exemple dans le brevet français 1 400 366 : 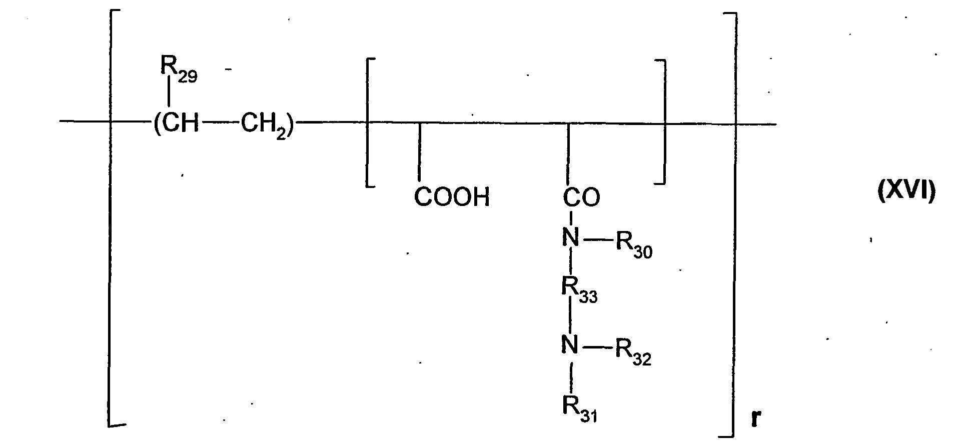 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂-, -CH₂-CH₂-CH₂-, -CH₂-CH(CH₃)-, R₃₁ ayant les significations mentionnées ci-dessus, ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- **(XVII)**

      où D désigne un radical 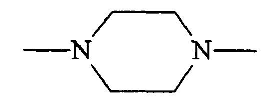 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- **(XVIII)**
   où D désigne un radical 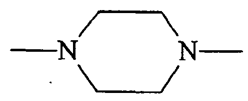 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.

Les compositions de l'invention comprennent de préférence un ou plusieurs tensioactifs qui peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO-)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL^{®} C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total de la composition.

Ladite composition peut également contenir des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 3% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi avec agent oxydant, l'agent oxydant est choisi de préférence parmi le, peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.
On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition [composition sans oxydant ou avec oxydant], est généralement compris entre les valeurs 2 et 12. Il est de préférence compris entre 3 et 11, et plus particulièrement compris entre 7 et 10. IL peut être ajusté à la valeur désirée au moyen d'agents acidifiants ou alcalinisants ou de tampons bien connus de l'état de la technique en teinture des fibres kératiniques.

Plus préférentiellement lorsque la composition contient un agent oxydant pour l'éclaircissement des fibres, le pH du mélange prêt à l'emploi est supérieur à 7 et encore plus préférentiellement supérieur à 8.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XIX) suivante : 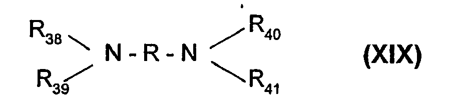 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Parmi les tampons, on peut citer ceux vendus par la société MERCK sous la marque TITRISOL, tels que que le tampon phosphate (KH₂PO₄ à 0,026 mole/l, Na₂HPO₄ à 0,041 mole/l), et le tampon borate (H₃BO₃ à 0,05 mole/l, KCI à 0,05 mole/l, NaOH à 0,022 mole/l).

Le procédé de teinture selon l'invention est conduit à température ambiante, et consiste à appliquer la composition de teinture prête à l'emploi selon l'invention (réalisée par mélange extemporané au moment de l'emploi de la composition de teinture et de la composition oxydante) sur les fibres kératiniques sèches ou humides, et à la laisser agir pendant un temps de pause variant de 5 secondes à 60 minutes environ, plus préférentiellement de 10 secondes à 5 minutes environ, et plus particulièrement de 30 secondes à 2 minutes environ, à rincer les fibres, puis éventuellement à les laver au shampooing, puis à les rincer à nouveau, et à les sécher.

Le procédé selon l'invention présente l'avantage de ne nécessiter que des temps d'application très courts, de l'ordre de 10 secondes à 5 minutes et plus particulièrement de 30 secondes à 2 minutes.

Il est possible de conduire le procédé à des températures plus élevées telles que celles produites par un casque de coiffure, 40°C environ, ou par un brushing, 70-80°C environ.

Des exemples concrets illustrant l'invention sont indiqués ci-après, sans pour autant présenter un caractère limitatif.

### EXEMPLES 1 et 2

On a préparé les compositions de teinture d'oxydation suivantes :

### [teneurs exprimées en grammes Matière Active (MA)]

| EXEMPLE | 1 | 2 |
|---|---|---|
| Paraphénylène diamine | 0,324 | 0,324 |
| Composé dicationique de formule (I)2 | 0,520 | |
| Composé dicationique de formule (I)1 | | 0,520 |
| Hydroxyéthylcellulose | 1,390 | 1,390 |
| Ammoniaque (40% NH₄OH) | 2,080 | 2,080 |
| Eau oxygénée 40 volumes | 60 | 60 |
| Eau déminéralisée q.s.p | 100 | 100 |

Chacune des compositions des exemples 1 et 2 a été appliquée, d'une part sur des mèches de cheveux gris naturels à 90% de blancs, d'autre part sur des mèches de cheveux gris permanentés à 90% de blancs, pendant 35 minutes à température ambiante (20°C).
A l'issue du temps de pause, les mèches de cheveux ont été rincées, puis séchées.
Avec les compositions des exemples 1 et 2, elles ont été teintes dans une nuance orangée pour l'exemple 1 et violine pour l'exemple 2, nuances par ailleurs chromatiques, peu sélectives et résistant bien aux lavages.

## Revendications

**1.** Composition de teinture pour fibres kératiniques humaines et plus particulièrement des cheveux, comprenant, dans un milieu approprié pour la teinture, au moins un colorant d'oxydation, **caractérisée par le fait qu'**elle comprend en outre au moins un composé dicationique choisi parmi ceux de formules (I), (II), ou (III), suivantes : 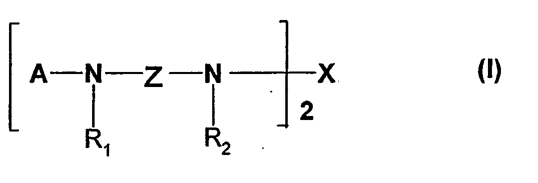 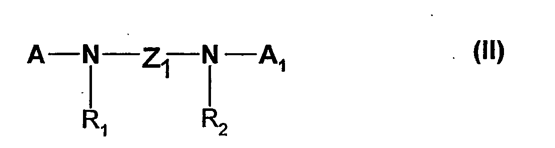 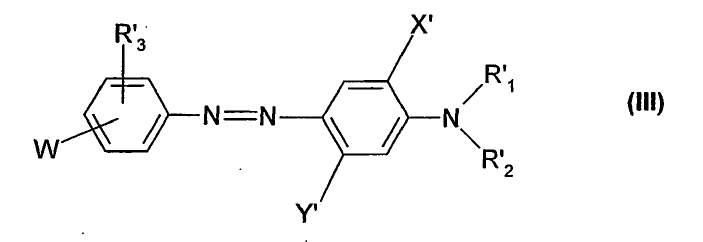 **formules (I) ou (II)** dans lesquelles:
- A et A1, indépendamment l'un de l'autre désignent un radical de formule (a) suivante 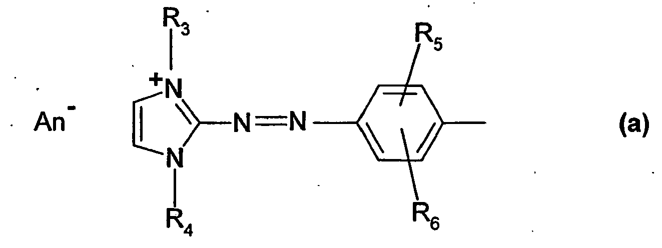
- Z désigne un radical aliphatique ou aromatique,
- Z₁ désigne un radical alkyle,
- R₁ et R₂, indépendamment l'un de l'autre, désignent un atome d'hydrogène, ou un radical (C₁-C₄)alkyl, ou (C₁-C₄)alkyl substitué par un ou plusieurs atomes d'halogène, un radical hydroxyl, carboxyl, cyano, un radical (C₁-C₄)alcoxy, un radical (C₁-C₄)alcoxy substitué par un ou plusieurs radicaux hydroxyl, ou (C₁-C₄)alcoxy, un radical amino, alkylamino, dialkylamino, aminocarbonyl, phényl, phénoxy ou phénylaminocarbonyl, dans lequel le radical phényl est non substitué ou substitué par un radical (C₁-C₄)alkyl, (C₁-C₄)alcoxy ou phénoxy,
ou encore R₁ et R₂ forment ensemble, avec les deux atomes d'azote qui les portent et le radical Z, un cycle pipérazinique,
- X est un radical de pontage choisi parmi : -CO- ; -CO-CH₂-CH₂-CO- ; -CO-CO- ; 1,4-dicarbonylphényl ; -CH₂-CH₂- ; ou une triazine de formules (b) ou (c) suivantes: 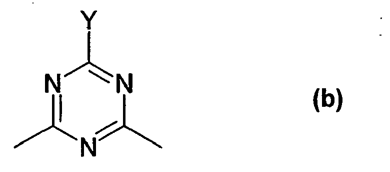
ou 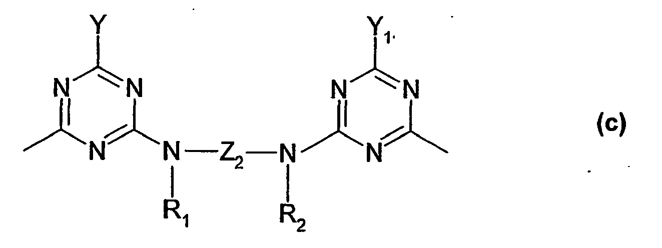 dans lesquelles :
Y et Y1, indépendamment l'un de l'autre désignent un atome d'halogène, ou un radical hydroxyl, ou amino, ou monoalkylamino, ou dialkylamino, ou 1-pipéridino, ou morpholino, ou 1-pipérazino, le radical pipérazino étant non substitué, ou substitué sur l'atome d'azote non attaché au cycle triazine par un radical (C₁-C₄)alkyl, lesdits radicaux alkyls étant non substitués ou substitués par hydroxyl, amino, mono-(C₁-C₄)alkylamino ou di-(C₁-C₄)alkylamino,
- Z₂ désigne un radical (C₂-C₈)alkylène ou forme, avec les deux atomes d'azote adjacents et les radicaux R₁ et R₂, un cycle pipérazinique,
- dans le radical de formule (a),
- R₃ et R₄, indépendamment l'un de l'autre, désignent un atome d'hydrogène, ou un radical (C₁-C₄)alkyl, ou (C₁-C₄)alkyl substitué par un ou plusieurs atomes d'halogène, un radical hydroxyl, carboxyl, cyano, un radical (C₁-C₄)alcoxy, un radical (C₁-C₄)alcoxy substitué par un radical hydroxyl ou (C₁-C₄)alcoxy un radical amino, alkylamino, dialkylamino, aminocarbonyl, phényl, phénoxy ou phénylaminocarbonyl, dans lequel le radical phényl est non substitué ou substitué par un radical (C₁-C₄)alkyl, (C₁-C₄)alcoxy ou phénoxy,
- R₅ et R₆, indépendamment l'un de l'autre, désignent un atome d'hydrogène, un radical (C₁-C₄)alkyl ou (C₁-C₄)alcoxy éventuellement substitués par un radical hydroxyl, carboxyl, halogène, cyano, (C₁-C₄)alcoxy éventuellement substitué par un radical hydroxyl ou (C₁-C₄)alcoxy, un radical amino, alkylamino, dialkylamino, aminocarbonyl, phényl, phénoxy ou phénylaminocarbonyl, dans lequel le radical phényl est non substitué
ou substitué par un radical (C₁-C₄)alkyl, (C₁-C₄)alcoxy ou phénoxy,
- An⁻ désigne un anion.
**formule (III)** dans laquelle,
- le nombre de charges cationiques est de deux,
- X' et Y', indépendamment l'un de l'autre, désignent hydrogène, halogène, (C₁-C₄)alkyl, (C₁-C₄)alcoxy, (C₁-C₄)alkylcarbonylamino, arylcarbonylamino, uréido, ou aryluréido,
- R'₁ désigne hydrogène, un radical alkyl ou aryl substitués, un radical alkyl ou aryl non substitués, ou la même désignation que R'₂
- R'₂ est un radical de formule (d) suivante: 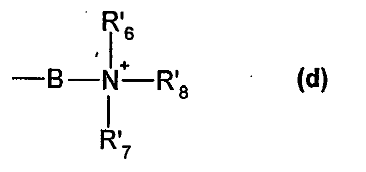
dans laquelle :
- B désigne un radical alkylène linéaire ou ramifié,
- R'₆ désigne hydrogène ou alkyl substitué ou non substitué,
- R'₇ et R'₈, indépendamment l'un de l'autre désignent alkyl substitué ou non substitué,
- R'₆ et R'₇, ensemble avec l'azote, forment un cycle à 5, 6, ou 7 chaînons, substitué ou non substitué, pouvant contenir d'autres hétéroatomes, ou bien R'₆ et R'₇ et R'₈ forment ensemble un cycle pyridinium,
- R'₃ désigne hydrogène, halogène, (C₁-C₄)alkyl, (C₁-C₄)alcoxy,
- W est un radical de formule (e) suivante : 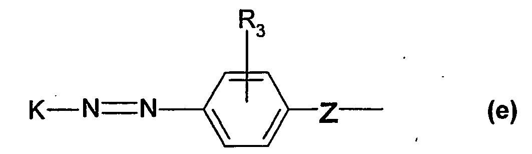
dans laquelle:
- K est un radical de couplage,
- Z désigne un radical de pontage choisi parmi les radicaux de formules :
-NR'₉-CO-;
-CO-NR'₉-NR'₉-CO-; 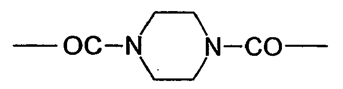 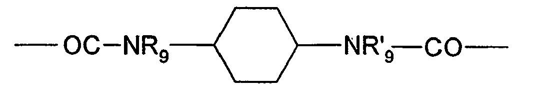 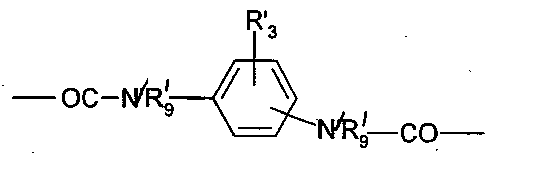 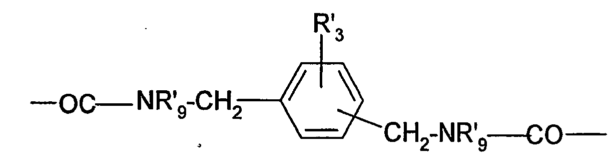
ou bien 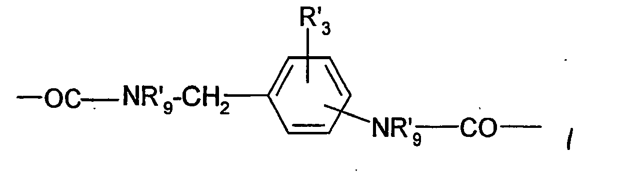 et dans lesquels R'₉ désigne, hydrogène, (C₂-C₄)alkylène non substitué ou substitué, le radical alkylène étant linéaire ou ramifié et pouvant être interrompu par un ou plusieurs groupements choisis parmi : -NR'9-, -O-, -S-.

**2.** Composition selon la revendication 1, **caractérisée par le fait que** dans la formule (I),
- R₁ et R₂, indépendamment l'un de l'autre, désignent hydrogène, (C₁-C₄)alkyl substitué par hydroxyl ou (C₁-C₄)alcoxy et plus particulièrement encore désignent hydrogène ou méthyle,
- Z désigne un radical alkyle en C₂-C₈ linéaire ou ramifié ou cyclique, éventuellement substitué par un hydroxy, alcoxy, halogène, la chaîne dudit radical étant éventuellement interrompue par un groupe -O- ou -NR₁- ; un radical 1,4-phényl, un radical 1,4-naphtyl éventuellement substitué par un alkyle, alcoxy, halogène ; Z pouvant formé avec R₁, R₂ et les 2 atomes d'azote, un cycle pipérazine, et préférentiellement,
- Z désigne un radical phényl non substitué, un radical phényl ou naphtyl substitué par 1
ou 2 radicaux méthyl ou méthoxy, un radical pipérazine par liaison avec R₁, R₂ et les 2 atomes d'azote, un radical (C₂-C₄)alkylène non subsitué ou substitué par un ou 2 hydroxyl,
- X désigne un groupement de formule (b).

**3.** Composition selon la revendication 1, **caractérisée par le fait que** dans la formule (II),
- Z1 désigne un radical alkyle en C₂-C₈ linéaire ou ramifié ou cyclique, éventuellement substitué par un hydroxy, alcoxy, halogène, la chaîne dudit radical étant éventuellement interrompue par un groupe -O- ou -NR₁- ; un cycle pipérazine formé avec R₁, R₂ et les deux atomes d'azote, et préférentiellement,
- Z₁ désigne un radical (C₂-C₆)alkylène non substitué ou substitué par un ou plusieurs hydroxyl, un cycle pipérazine formé avec R₁, R₂ et les deux atomes d'azote ; et plus particulièrement encore un radical (C₂-C₄)alkylène non substitué,
- R₃ et R₄ désignent méthyl ou éthyl, et R₅ et R₆ désignent hydrogène, méthyl, ou méthoxy.

**4.** Composition selon la revendication 1, **caractérisée par le fait que** dans la formule (III),
B désigne éthylène, n-propylène, isopropylène ou n-butylène,
K désigne un composé de couplage choisi parmi ceux de formule (f), (g) ou (h) suivantes 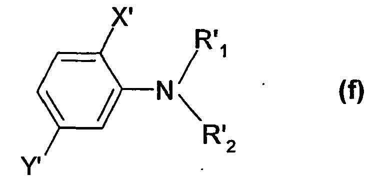 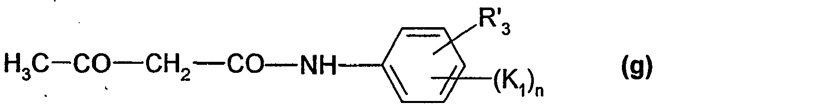 ou 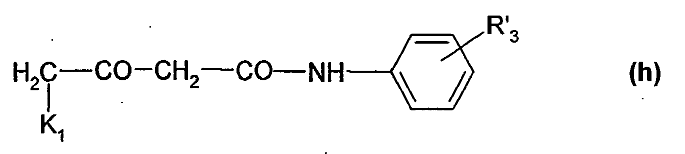 dans lesquelles,
- X', Y' et R'₁ et R'₂, ont la même désignation que dans la formule (III),
- n est égal à 1 ou 2,
- K₁ désigne le radical de formule :

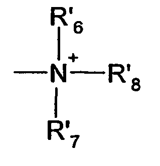

**5.** Composition selon l'une quelconque des revendications 1 ou 2, **caractérisée par le fait que** le composé de formule (I) ou de formule (II) est choisi parmi ceux de formules (I)1 à (I)7 suivantes :

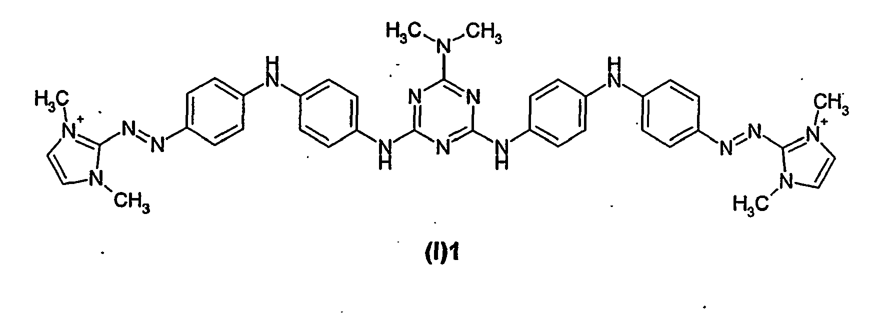

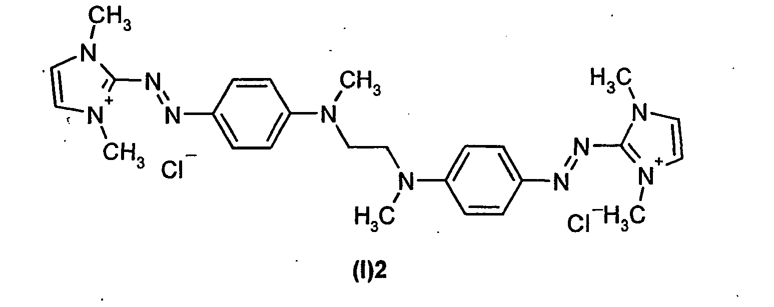

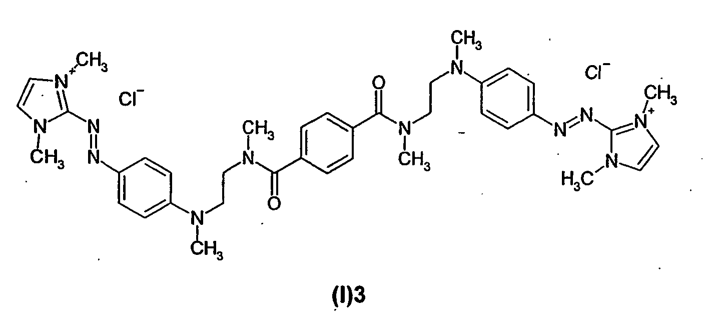

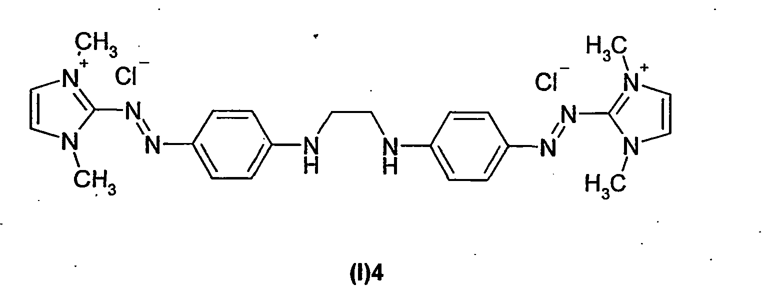

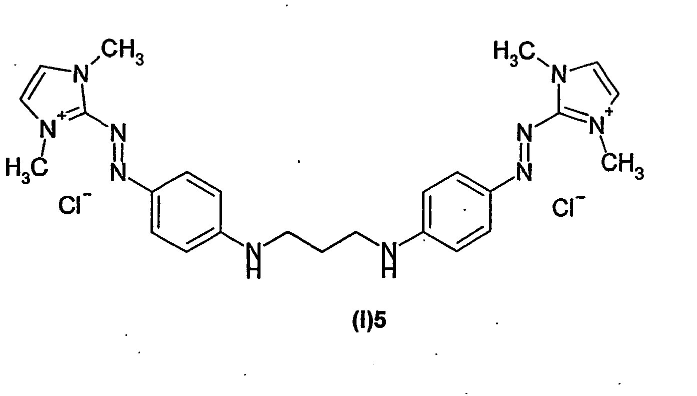

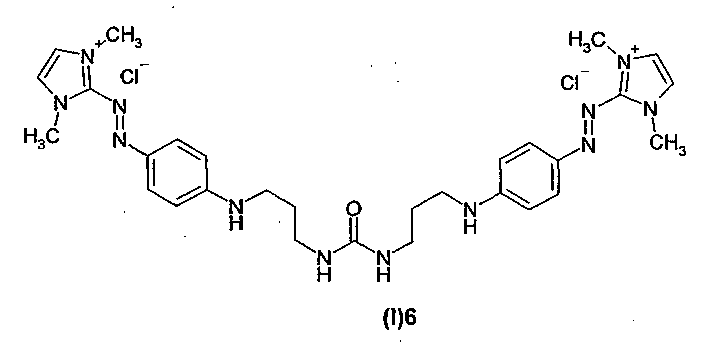

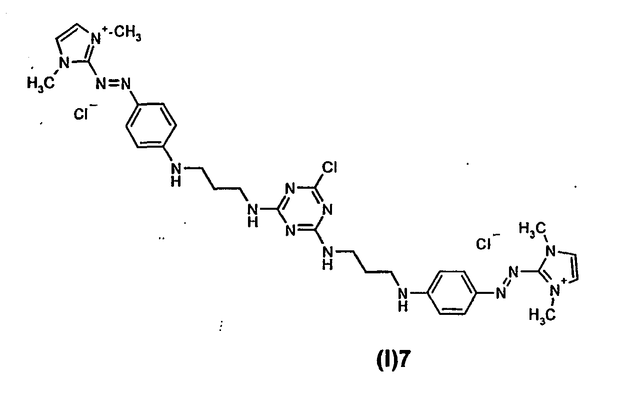

**6.** Composition selon l'une quelconque des revendications 1 ou 4, **caractérisée par le fait que** le composé de formule (III) est le composé suivant :

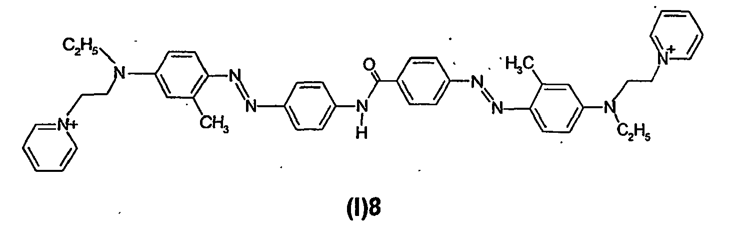

**7.** Composition selon l'une quelconque des revendications précédentes, **caractérisée par le fait que** le ou les colorants directs de formules (I), (II), ou (III) sont présents en une quantité variant de 0,01 à 40% en poids par rapport au poids total de la composition.

**8.** Composition selon la revendication 7, **caractérisée par le fait que** le ou les colorants directs de formules (I), (II), ou (III) sont présents en une quantité variant de 0,1 à 20% en poids par rapport au poids total de la composition.

**9.** Composition selon l'une quelconque des revendications précédentes, **caractérisée par le fait que** le colorant d'oxydation est choisi parmi les bases d'oxydation et/ou les coupleurs.

**10.** Composition selon la revendication 9, **caractérisée par le fait qu'**elle contient au moins une base d'oxydation.

**11.** Composition selon les revendications 9 ou 10, **caractérisée par le fait que** les bases d'oxydation sont choisies parmi les ortho- ou para- phénylènediamines, les bases doubles, les ortho- ou para- aminophénols, et les bases hétérocycliques, ainsi que les sels d'addition de ces composés avec un acide.

**12.** Composition selon la revendication 11, **caractérisée par le fait que** les paraphénylènediamines sont choisies parmi les composés de structure (V) suivante : 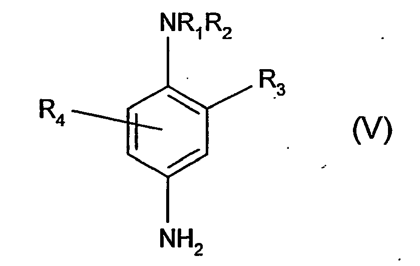 dans laquelle :
R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un radical alkyle en C₁-C₄.

**13.** Composition selon la revendication 11, **caractérisée par le fait que** les bases doubles sont choisies parmi les composés de structure (VI) suivante : 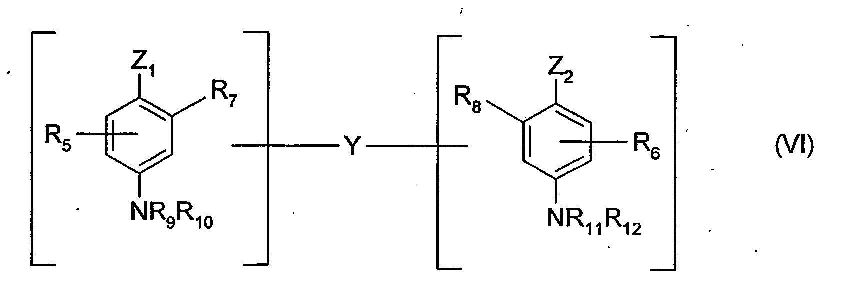 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C4 ; étant entendu que les composés de formule (VI) ne comportent qu'un seul bras de liaison Y par molécule.

**14.** Composition selon la revendication 11, **caractérisée par le fait que** les paraaminophénols sont choisis parmi les composés de structure (VII) suivante : 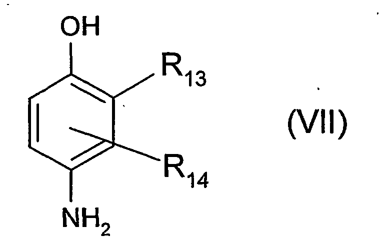 dans laquelle :
**R₁₃** représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
**R₁₄** représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

**15.** Composition selon la revendication 11, **caractérisée par le fait que** les bases hétérocycliques sont choisies parmi les dérivés pyridiniques, les dérivés pyrimidiniques dont les pyrazolopyrimidines, les dérivés pyrazoliques.

**16.** Composition selon l'une quelconque des revendications 11 à 15, **caractérisée par le fait que** les bases d'oxydation sont présentes dans des concentrations allant de 0,0005 à 12% en poids par rapport au poids total de la composition.

**17.** Composition selon la revendication 9, **caractérisée par le fait que** les coupleurs sont choisis parmi les métaphénylènediamines, les méta-aminophénols, les métadiphénols, les coupleurs hétérocycliques, et les sels d'addition de ces composés avec un acide.

**18.** Composition selon l'une quelconque des revendications 9 ou 17, **caractérisée par le fait que** les coupleurs sont présents dans des concentrations allant de 0,0001 à 10% en poids par rapport au poids total de la composition.

**19.** Composition selon l'une quelconque des revendications 11 à 17, **caractérisée par le fait que** les sels d'addition avec un acide des colorants d'oxydation sont choisis parmi les chlorhydrates, les bromhydrates, les sulfates, les tartrates, les lactates et les acétates.

**20.** Composition selon l'une quelconque des revendications précédentes, **caractérisée par le fait qu'**elle contient en outre au moins, dans la proportion de 0,01 à 10% en poids par rapport au poids total de la composition, d'un polymère cationique ou amphotère choisi parmi :
-1/ les homopolymères de chlorure de diméthyldiallylammonium;
-2/ les polymères au motifs récurrents de formules (W) ou (U) suivantes : 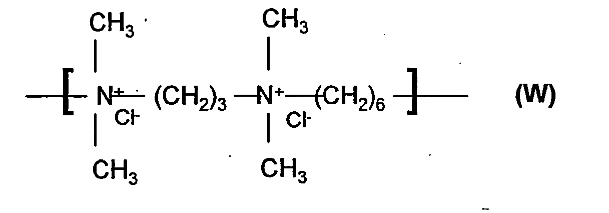 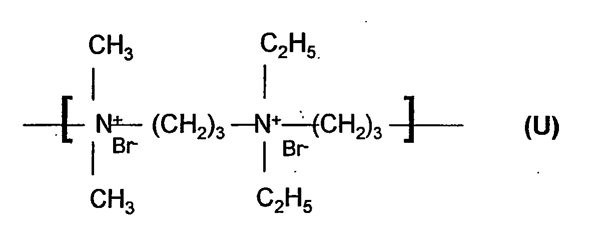
-3/ les polymères aux motifs de formule (IX) suivante : 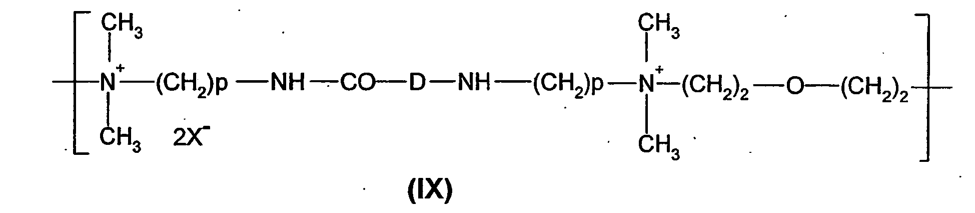 pour laquelle, p est égal à 3, et,
a) D désigne la valeur zéro, X désigne un atome de chlore,
b) D représente un groupement -(CH₂)₄ -CO-, X désigne un atome de chlore,
c) D représente un groupement -(CH₂)₇ -CO -, X désigne un atome de chlore,
d) un " Block Copolymer " formé de motifs correspondant aux polymères décrits aux alinéas a) et b);
-4/ les copolymères d'acide acrylique et de chlorure de diméthyldiallylammonium.

**21.** Composition selon l'une quelconque des revendications précédentes, **caractérisée par le fait qu'**elle contient au moins un tensioactif choisi parmi les tensioactifs anioniques, cationiques, non ioniques ou amphotères.

**22.** Composition selon l'une quelconque des revendications précédentes, **caractérisée par le fait qu'**elle contient en outre au moins un agent réducteur, dans des quantités allant de 0,05 à 3% en poids par rapport au poids total de la composition.

**23.** Procédé de teinture d'oxydation des fibres kératiniques humaines et en particulier des cheveux, consistant à appliquer sur les fibres sèches ou humides, à température ambiante, une composition de teinture prête à l'emploi, réalisée par mélange extemporané au moment de l'emploi d'une composition de teinture contenant, dans un milieu approprié pour la teinture, au moins un colorant d'oxydation et au moins un composé dicationique de formules (I), (II), ou (III), telles que définies selon l'une quelconque des revendications 1 à 6 et d'une composition oxydante, et à la laisser agir pendant un temps de pause variant de 5 secondes à 60 minutes, à rincer les fibres, puis éventuellement à les laver au shampooing, puis à les rincer à nouveau, et à les sécher.

**24.** Procédé selon la revendication 23, **caractérisé par le fait que** le temps de pause varie de 10 secondes à 5 minutes et plus particulièrement de 30 secondes à 2 minutes.

**25.** Dispositif pour la teinture d'oxydation des fibres kératiniques humaines et en particulier des cheveux, qui comprend un premier compartiment renfermant, dans un milieu approprié pour la teinture, au moins un colorant d'oxydation et au moins un composé dicationique de formules (I), (II), ou (III), telles que définies selon l'une quelconque des revendications 1 à 6 et un deuxième compartiment renfermant un agent oxydant.

## Claims

1. Dye composition for human keratin fibres, and more particularly the hair, comprising at least one oxidation dye in a medium that is suitable for dyeing, **characterized in that** it also comprises at least one dicationic compound chosen from those of formula (I), (II) or (III) below: 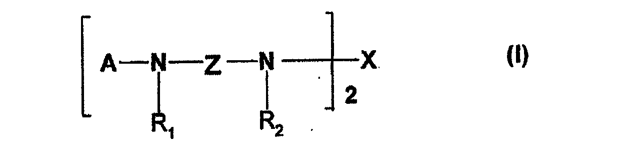 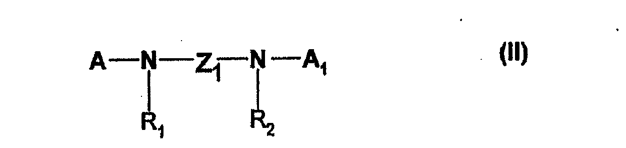 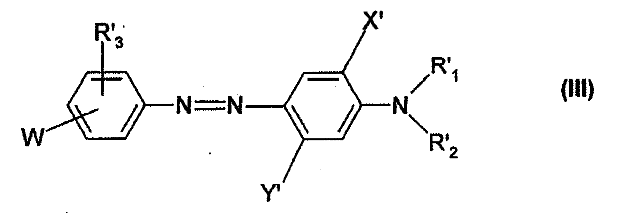 in which formula (I) or (II):
- A and A1, independently of each other, denote a radical of formula (a) below 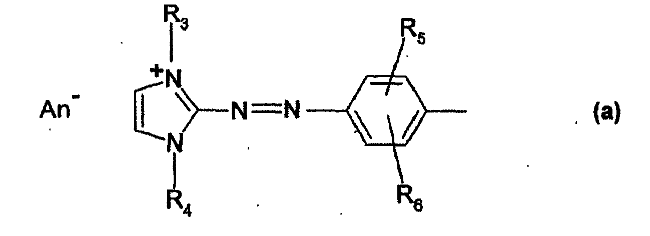
- Z denotes an aliphatic or aromatic radical,
- Z₁ denotes an alkyl radical,
- R₁ and R₂, independently of each other, denote a hydrogen atom or a (C₁-C₄)alkyl radical, or a (C₁-C₄)alkyl radical substituted with one or more halogen atoms, a hydroxyl, carboxyl or cyano radical, a (C₁-C₄)alkoxy radical, a (C₁-C₄)alkoxy radical substituted with one or more hydroxyl or (C₁-C₄)alkoxy radicals, or an amino, alkylamino, dialkylamino, aminocarbonyl, phenyl, phenoxy or phenylaminocarbonyl radical, in which the phenyl radical is unsubstituted or substituted with a (C₁-C₄) alkyl, (C₁-C₄) alkoxy or phenoxy radical,
or R₁ and R₂ form, together with the two nitrogen atoms that bear them and the radical Z, a piperazine ring, - X is a bridging radical chosen from: -CO-; -CO-CH₂-CH₂-CO-; -CO-CO-; 1,4-dicarbonylphenyl; -CH₂-CH₂-; or a triazine of formula (b) or (c) below: 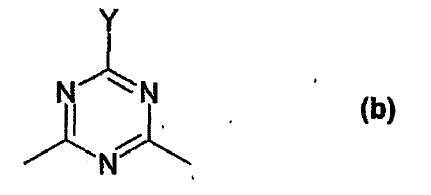 or 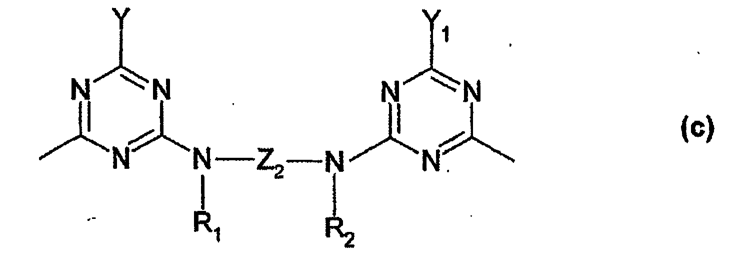
in which:
Y and Y1, independently of each other, denote a halogen atom or a hydroxyl, amino, monoalkylamino, dialkylamino, 1-piperidino, morpholino or 1-piperazino radical, the piperazino radical being unsubstituted or substituted on the nitrogen atom not attached to the triazine ring with a (C₁-C₄)alkyl radical, said alkyl radicals being unsubstituted or substituted with hydroxyl, amino, mono(C₁-C₄) alkylamino or di(C₁-C₄)-alkylamino,
- Z₂ denotes a (C₂-C₈)alkylene radical or forms a piperazine ring with the two adjacent nitrogen atoms and the radicals R₁ and R₂,
- in the radical of formula (a),
- R₃ and R₄, independently of each other, denote a hydrogen atom or a (C₁-C₄)alkyl radical, or (C₁-C₄)alkyl substituted with one or more halogen atoms, a hydroxyl, carboxyl, or cyano radical, a (C₁-C₄)alkoxy radical, a (C₁-C₄)alkoxy radical substituted with a hydroxyl or (C₁-C₄)alkoxy radical, or an amino, alkylamino, dialkylamino, aminocarbonyl, phenyl, phenoxy or phenylaminocarbonyl radical, in which the phenyl radical is unsubstituted or substituted with a (C₁-C₄)-alkyl, (C₁-C₄)alkoxy or phenoxy radical,
- R₅ and R₆, independently of each other, denote a hydrogen atom, a (C₁-C₄)alkyl or (C₁-C₄)alkoxy radical optionally substituted with a hydroxyl, carboxyl, halogen, cyano , (C₁-C₄)alkoxy radical optionally substituted with a hydroxyl or (C₁-C₄)alkoxy radical, or an amino, alkylamino, dialkylamino, aminocarbonyl, phenyl, phenoxy or phenylaminocarbonyl radical, in which the phenyl radical is unsubstituted or substituted with a (C₁-C₄) alkyl, (C₁-C₄) alkoxy or phenoxy radical,
- An⁻ denotes an anion;
in which formula (III),
- the number of cationic charges is two,
- X' and Y', independently of each other, denote hydrogen, halogen, (C₁-C₄) alkyl, (C₁-C₄) alkoxy, (C₁-C₄)-alkylcarbonylamino, arylcarbonylamino, ureido or arylureido,
- R'₁ denotes hydrogen, a substituted alkyl or aryl radical, an unsubstituted alkyl or aryl radical, or the same meaning as R'₂
- R'₂ is a radical of formula (d) below: 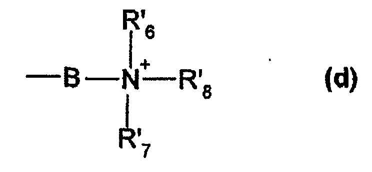
in which:
- B denotes a linear or branched alkylene radical,
- R'₆ denotes hydrogen or substituted or unsubstituted alkyl,
- R'₇ and R'₈, independently of each other, denote substituted or unsubstituted alkyl,
- R'₆ and R'₇, together with the nitrogen, form a substituted or unsubstituted 5-, 6- or 7-membered ring, which may contain other hetero atoms, or alternatively R'₆, R'₇ and R'₈ together form a pyridinium ring,
- R'₃ denotes hydrogen, halogen, (C₁-C₄)alkyl or (C₁-C₄)-alkoxy,
- W is a radical of formula (e) below: 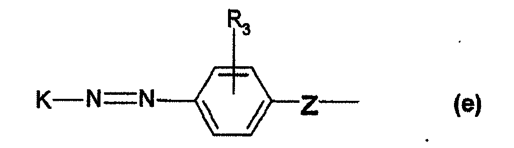
in which:
- K is a coupling radical,
- Z denotes a bridging radical chosen from the radicals of formulae:
- **NR'₉-CO-;**
- **CO-NR'₉-NR'₉-CO-;** 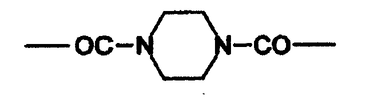 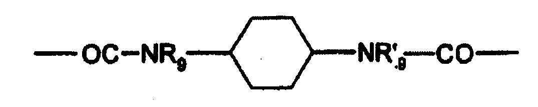 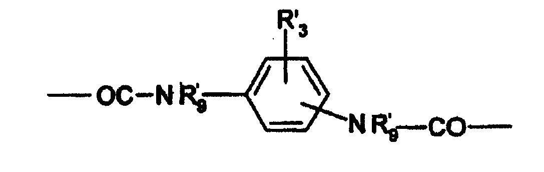 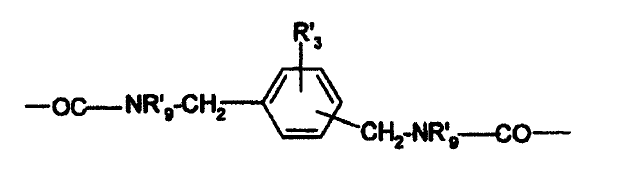 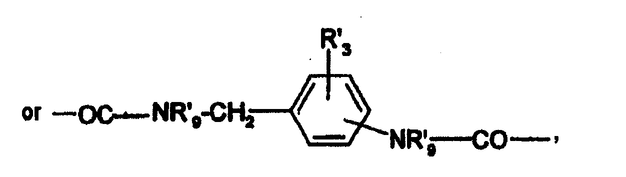 and in which R'₉ denotes hydrogen or unsubstituted or substituted (C₂-C₄)alkylene, the alkylene radical being linear or branched and possibly being interrupted with one or more groups chosen from: -NR'9-, -O- and -S-.

2. Composition according to Claim 1,
**characterized in that,** in formula (I),
- R₁ and R₂, independently of each other, denote hydrogen, (C₁-C₄)alkyl substituted with hydroxyl or (C₁-C₄)alkoxy, and even more particularly denote hydrogen or methyl,
- Z denotes a linear, branched or cyclic C₂-C₈ alkyl radical optionally substituted with a hydroxyl, alkoxy or halogen, the chain of said radical optionally being interrupted with a group -O- or -NR₁-; a 1,4-phenyl radical, a 1,4-naphthyl radical optionally substituted with an alkyl, alkoxy or halogen; Z possibly forming a piperazine ring with R₁, R₂ and the 2 nitrogen atoms, and preferably
- Z denotes an unsubstituted phenyl radical, a phenyl or naphthyl radical substituted with 1 or 2 methyl or methoxy radicals, a piperazine radical by bonding with R₁, R₂ and the 2 nitrogen atoms, or a (C₂-C₄)alkylene radical which is unsubstituted or substituted with one or two hydroxyls,
- X denotes a group of formula (b).

3. Composition according to Claim 1,
**characterized in that,** in formula (II),
- Z₁ denotes a linear, branched or cyclic C₂-C₈ alkyl radical, optionally substituted with a hydroxyl, alkoxy or halogen, the chain of said radical optionally being interrupted with a group -O- or -NR₁-; a piperazine ring formed with R₁, R₂ and the two nitrogen atoms, and preferably
- Z₁ denotes a (C₂-C₆) alkylene radical which is unsubstituted or substituted with one or more hydroxyls, a piperazine ring formed with R₁, R₂ and the two nitrogen atoms; and even more particularly an unsubstituted (C₂-C₄)alkylene radical,
- R₃ and R₄ denote methyl or ethyl, and R₅ and R₆ denote hydrogen, methyl or methoxy.

4. Composition according to Claim 1,
**characterized in that,** in formula (III),
B denotes ethylene, n-propylene, isopropylene or n-butylene,
K denotes a coupling compound chosen from those of formula (f), (g) or (h) below 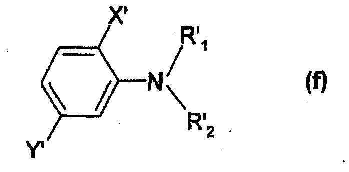 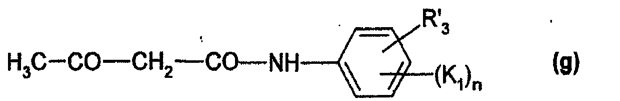 or 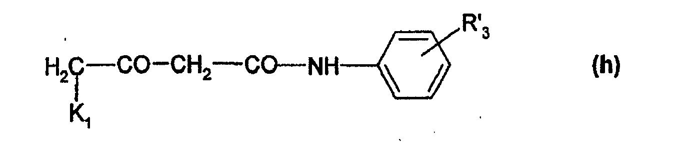 in which,
- X', Y' and R'₁ and R'₂ have the same meaning as in formula (III),
- n is equal to 1 or 2,
- K₁ denotes the radical of formula:

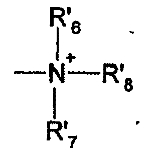

5. Composition according to either of Claims 1 and 2, **characterized in that** the compound of formula (I) or of formula (II) is chosen from those of formulae (I)1 to (1)7 below:

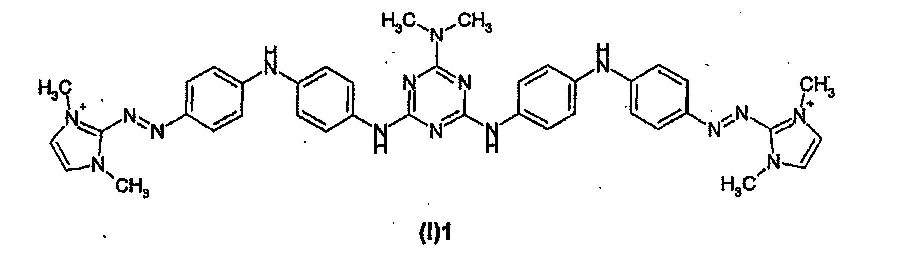

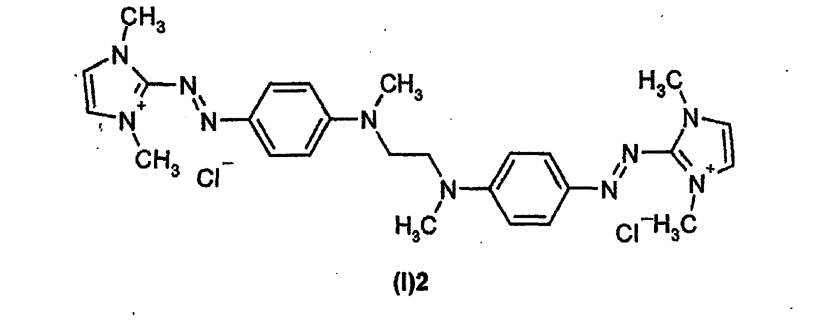

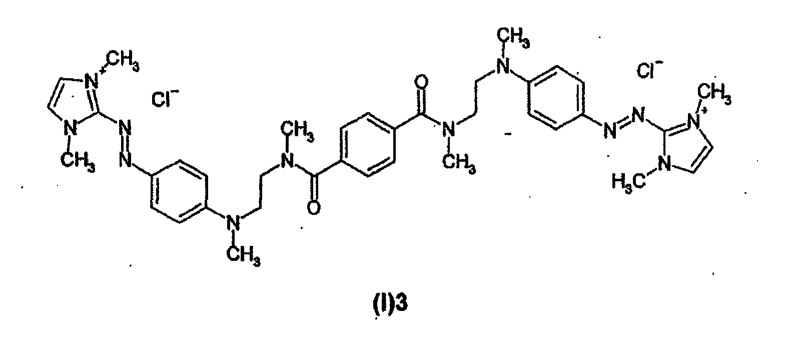

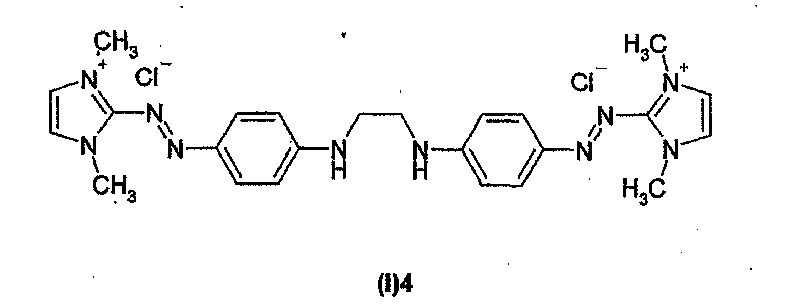

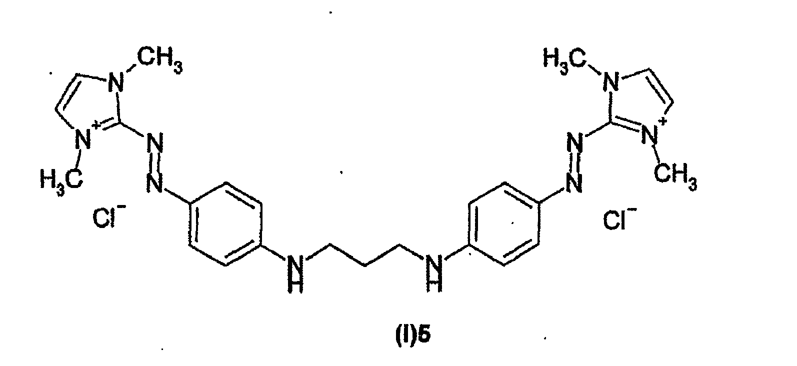

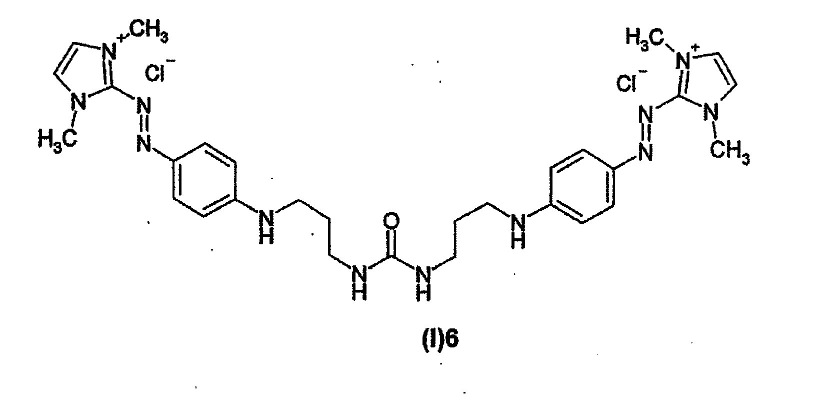

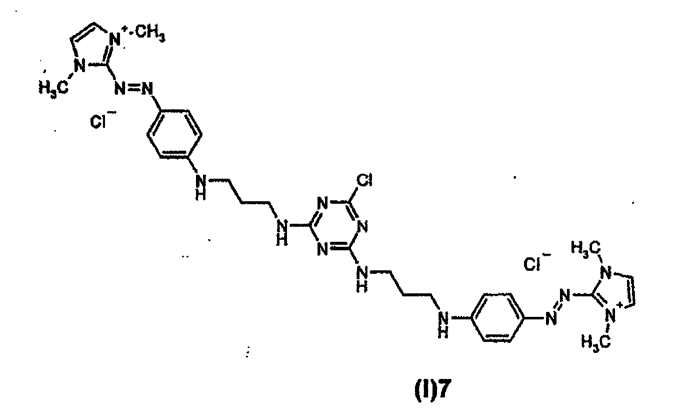

6. Composition according to either of Claims 1 and 4, **characterized in that** the compound of formula (III) is the following compound:

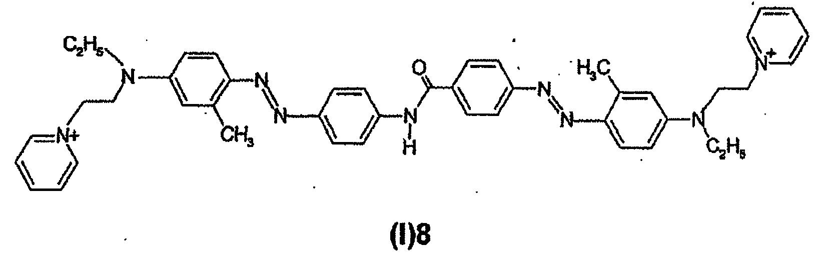

7. Composition according to any one of the preceding claims, **characterized in that** the direct dye(s) of formula (I), (II) or (III) is (are) present in an amount ranging from 0.01% to 40% by weight relative to the total weight of the composition.

8. Composition according to Claim 7,
**characterized in that** the direct dye(s) of formula (I), (II) or (III) is (are) present in an amount ranging from 0.1% to 20% by weight relative to the total weight of the composition.

9. Composition according to any one of the preceding claims, **characterized in that** the oxidation dye is chosen from oxidation bases and/or couplers.

10. Composition according to Claim 9,
**characterized in that** it contains at least one oxidation base.

11. Composition according to Claim 9 or 10, **characterized in that** the oxidation bases are chosen from ortho- or para-phenylenediamines, double bases, ortho- or para-aminophenols and heterocyclic bases, and also the addition salts of these compounds with an acid.

12. Composition according to Claim 11,
**characterized in that** the para-phenylenediamines are chosen from the compounds of structure (V) below: 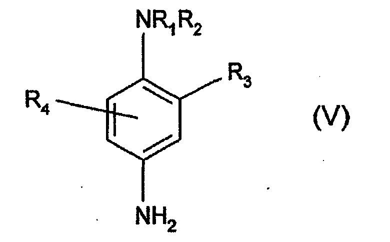 in which:
- R₁ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical, a C₁-C₄ alkyl radical substituted with a nitrogenous group, a phenyl radical or a 4'-aminophenyl radical;
- R₂ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical or a C₁-C₄ alkyl radical substituted with a nitrogenous group;
- R₁ and R₂ may also form with the nitrogen atom that bears them a 5- or 6-membered nitrogenous heterocycle optionally substituted with one or more alkyl, hydroxy or ureido groups;
- R₃ represents a hydrogen atom, a halogen atom such as a chlorine atom, a C₁-C₄ alkyl radical, a sulfo radical, a carboxy radical, a C₁-C₄ monohydroxyalkyl radical, a C₁-C₄ hydroxyalkoxy radical, an acetylamino (C₁-C₄) alkoxy radical, a C₁-C₄ mesylaminoalkoxy radical or a carbamoylamino(C₁-C₄)alkoxy radical,
- R₄ represents a hydrogen or halogen atom or a C₁-C₄ alkyl radical.

13. Composition according to Claim 11,
**characterized in that** the double bases are chosen from the compounds of structure (VI) below: 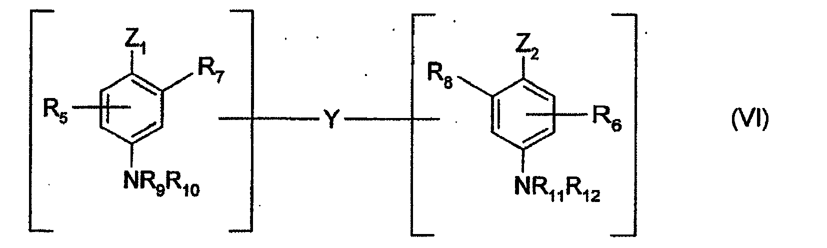 in which:
- Z₁ and Z₂, which may be identical or different, represent a hydroxyl or -NH₂ radical which may be substituted with a C₁-C₄ alkyl radical or with a linker arm Y;
- the linker arm Y represents a linear or branched alkylene chain containing from 1 to 14 carbon atoms, which may be interrupted by or terminated with one or more nitrogenous groups and/or one or more hetero atoms such as oxygen, sulfur or nitrogen atoms, and optionally substituted with one or more hydroxyl or C₁-C₆ alkoxy radicals;
- R₅ and R₆ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm Y;
- R₇, R₈, R₉, R₁₀, R₁₁ and R₁₂, which may be identical or different, represent a hydrogen atom, a linker arm Y or a C₁-C₄ alkyl radical; it being understood that the compounds of formula (VI) contain only one linker arm Y per molecule.

14. Composition according to Claim 11, **characterized in that** the para-aminophenols are chosen from the compounds of structure (VII) below: 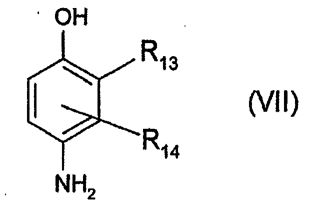 in which:
- R₁₃ represents a hydrogen atom, a halogen atom such as fluorine, a C₁-C₄ alkyl, C₁-C₄ monohydroxyalkyl, (C₁-C₄)-alkoxy(C₁-C₄) alkyl, C₁-C₄ aminoalkyl or hydroxy(C₁-C₄)alkylamino(C₁-C₄)alkyl radical,
- R₁₄ represents a hydrogen atom or a halogen atom such as fluorine, a C₁-C₄-alkyl, C₁-C₄ monohydroxyalkyl, C₂-C₄ polyhydroxyalkyl, C₁-C₄ aminoalkyl, C₁-C₄ cyanoalkyl or (C₁-C₄)alkoxy(C₁-C₄)alkyl radical.

15. Composition according to Claim 11, **characterized in that** the heterocyclic bases are chosen from pyridine derivatives, pyrimidine derivatives including pyrazolopyrimidines, and pyrazole derivatives.

16. Composition according to any one of Claims 11 to 15, **characterized in that** the oxidation bases are present in concentrations ranging from 0.0005% to 12% by weight relative to the total weight of the composition.

17. Composition according to Claim 9, **characterized in that** the couplers are chosen from meta-phenylenediamines, meta-aminophenols, meta-diphenols and heterocyclic couplers, and the addition salts of these compounds with an acid.

18. Composition according to either of Claims 9 and 17, **characterized in that** the couplers are present in concentrations ranging from 0.0001% to 10% by weight relative to the total weight of the composition.

19. Composition according to any one of Claims 11 to 17, **characterized in that** the addition salts with an acid of the oxidation dyes are chosen from the hydrochlorides, hydrobromides, sulfates, tartrates, lactates and acetates.

20. Composition according to any one of the preceding claims, **characterized in that** it also contains, in a proportion of from 0.01% to 10% by weight relative to the total weight of the composition, at least one cationic or amphoteric polymer chosen from:
-1/ dimethyldiallylammonium chloride homopolymers;
-2/ polymers containing repeating units of formula (W) or (U) below: 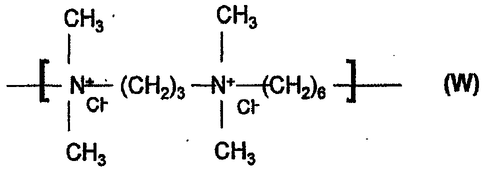 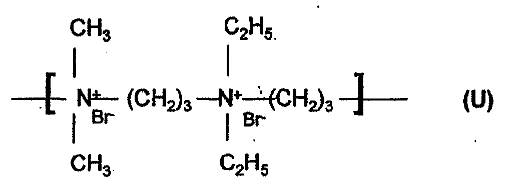
-3/ polymers containing units of formula (IX) below: 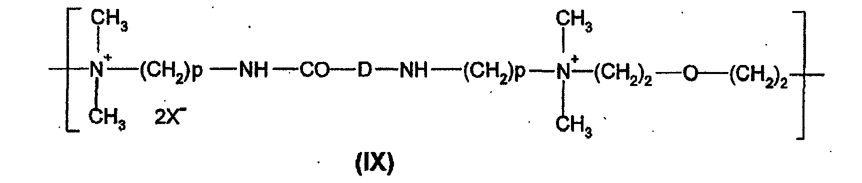 for which p is equal to 3, and
a) D denotes the value zero, X denotes a chlorine atom,
b) D represents a -(CH₂)₄-CO- group, X denotes a chlorine atom,
c) D represents a -(CH₂)₇-CO- group, X denotes a chlorine atom,
d) a "Block Copolymer" formed from units corresponding to the polymers described in paragraphs a) and b);
-4/ copolymers of acrylic acid and of dimethyldiallylammonium chloride.

21. Composition according to any one of the preceding claims, **characterized in that** it contains at least one surfactant chosen from anionic, cationic, nonionic and amphoteric surfactants.

22. Composition according to any one of the preceding claims, **characterized in that** it also contains at least one reducing agent, in amounts ranging from 0.05% to 3% by weight relative to the total weight of the composition.

23. Process for the oxidation dyeing of human keratin fibres, and in particular the hair, which consists in applying a ready-to-use dye composition, prepared by extemporaneously mixing together a dye composition containing, in a medium that is suitable for dyeing, at least one oxidation dye and at least one dicationic compound of formula (I), (II) or (III), as defined according to any one of Claims 1 to 6, and an oxidizing composition at the time of use, onto wet or dry fibres, at room temperature, and in leaving it to act for a leave-in time ranging from 5 seconds to 60 minutes, rinsing the fibres and then optionally washing them with shampoo, followed by rinsing them again and drying them.

24. Process according to Claim 23, **characterized in that** the leave-in time ranges from 10 seconds to 5 minutes and more particularly from 30 seconds to 2 minutes.

25. Device for the oxidation dyeing of human keratin fibres, and in particular the hair, which comprises a first compartment containing, in a medium that is suitable for dyeing, at least one oxidation dye and at least one dicationic compound of formula (I), (II) or (III), as defined according to any one of Claims 1 to 6, and a second compartment containing an oxidizing agent.

## Patentansprüche

1. Zusammensetzung zum Färben von menschlichen Keratinfasern und insbesondere Haaren, die in einem zum Färben geeigneten Medium mindestens einen Oxidationsfarbstoff enthält, **dadurch gekennzeichnet, dass** sie ferner mindestens eine dikationische Verbindung enthält, die unter den Verbindungen der folgenden Formeln (I), (II) oder (III) ausgewählt ist: 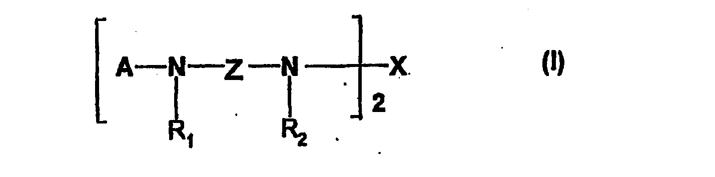 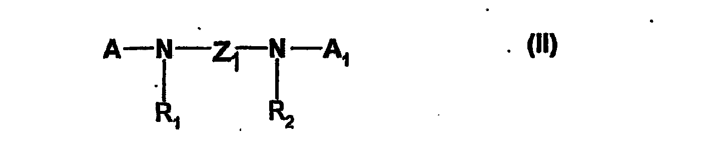 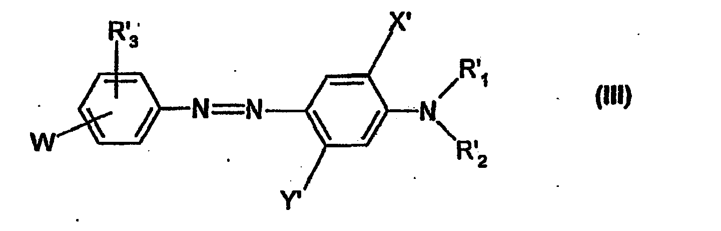 in den Formeln (I) oder (II):
- A und A1 bedeuten unabhängig voneinander eine Gruppe der folgenden Formel (a): 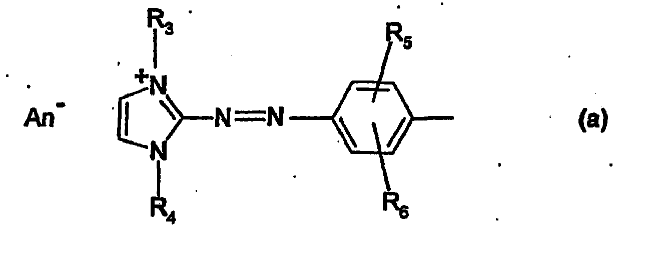
- Z ist eine aliphatische oder aromatische Gruppe,
- Z₁ bedeutet eine Alkylgruppe,
- R₁ und R₂ bedeuten unabhängig voneinander ein Wasserstoffatom, eine (C₁₋₄)Alkylgruppe oder eine (C₁₋₄)Alkylgruppe, die substituiert ist mit einem oder mehreren Halogenatomen, Hydroxy, Carboxy, Cyano, (C₁₋₄)Alkoxy, (C₁₋₄)Alkoxy, das mit einer oder mehreren Hydroxygruppen oder (C₁₋₄)Alkoxy substituiert ist, Amino, Alkylamino, Dialkylamino, Aminocarbonyl, Phenyl, Phenoxy oder Phenylaminocarbonyl, bei dem die Phenylgruppe unsubstituiert vorliegt oder mit einer Gruppe (C₁₋₄)Alkyl, (C₁₋₄)Alkoxy oder Phenoxy substituiert ist, oder die Gruppen R₁ oder R₂ bilden gemeinsam mit den beiden Stickstoffatomen, von denen sie getragen werden, und der Gruppe Z einen Piperazinring,
- X ist eine Verbindungsgruppe, die ausgewählt ist unter: -CO-; -CO-CH₂-CH₂-CO-; -CO-CO; 1,4-Dicarbonylphenyl; -CH₂-CH₂-; oder ein Triazin der folgenden Formeln (b) oder (c): 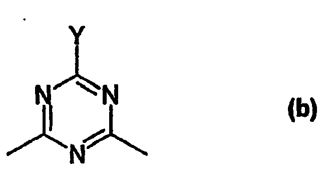 oder 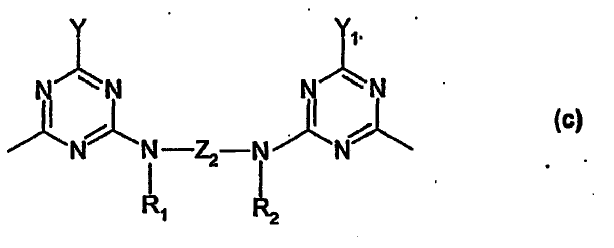 in den Formeln:
- Y und Y1 bedeuten unabhängig voneinander ein Halogenatom oder eine Gruppe Hydroxy oder Amino oder Monoalkylamino oder Dialkylamino oder 1-Piperidino oder Morpholino oder 1-Piperazino, wobei die Piperazinogruppe unsubstituiert vorliegt oder am Stickstoffatom, das nicht an den Triazinring gebunden ist, mit einer (C₁₋₄)Alkylgruppe substituiert ist, wobei die Alkylgruppen unsubstituiert vorliegen oder mit Hydroxy, Amino, Mono-(C₁₋₄)alkylamino oder Di-(C₁₋₄)alkylamino substituiert sind,
- Z₂ ist eine Gruppe (C₂₋₈)Alkylen oder bildet mit den beiden angrenzenden Stickstoffatomen und den Gruppen R₁ und R₂ einen Piperazinring;
- in der Gruppe der Formel (a):
- R₃ und R₄ bedeuten unabhängig voneinander ein Wasserstoffatom oder eine (C₁₋₄)Alkylgruppe oder eine (C₁₋₄)Alkylgruppe, die substituiert ist mit einem oder mehreren Halogenatomen, Hydroxy, Carboxy, Cyano, (C₁₋₄)Alkoxy, (C₁₋₄)Alkoxy, das mit einer Gruppe Hydroxy oder (C₁₋₄)Alkoxy substituiert ist, Amino, Alkylamino, Dialkylamino, Aminocarbonyl, Phenyl, Phenoxy oder Phenylaminocarbonyl, bei dem die Phenylgruppe unsubstituiert vorliegt oder mit einer Gruppe (C₁₋₄) Alkyl, (C₁₋₄)Alkoxy oder Phenoxy substituiert ist,
- R₅ und R₆ bedeuten unabhängig voneinander ein Wasserstoffatom, eine (C₁₋₄)Alkylgruppe oder eine (C₁₋₄)Alkoxygruppe, die gegebenenfalls substituiert sind mit einer Gruppe Hydroxy, Carboxy, Halogen, Cyano, (C₁₋₄)Alkoxy, das gegebenenfalls mit einer Gruppe Hydroxy oder (C₁₋₄)Alkoxy substituiert ist, Amino, Alkylamino, Dialkylamino, Aminocarbonyl, Phenyl, Phenoxy oder Phenylaminocarbonyl, bei dem die Phenylgruppe unsubstituiert vorliegt oder mit einer Gruppe (C₁₋₄)Alkyl, (C₁₋₄)Alkoxy oder Phenoxy substituiert ist,
- An⁻ bedeutet ein Anion;
in der Formel (III):
- die Anzahl der kationischen Ladungen beträgt zwei,
- X' und Y' bedeuten unabhängig voneinander Wasserstoff, Halogen, (C₁₋₄)Alkyl, (C₁₋₄)Alkoxy, (C₁₋₄)Alkylcarbonylamino, Arylcarbonylamino, Ureido oder Arylureido,
- R'₁ bedeutet Wasserstoff, eine Alkylgruppe oder eine Arylgruppe, die substituiert sind, eine Alkylgruppe oder Arylgruppe, die unsubstituiert vorliegen, oder die Bedeutung, die für R'₂ angegeben ist,
- R'₂ ist eine Gruppe der folgenden Formel (d): 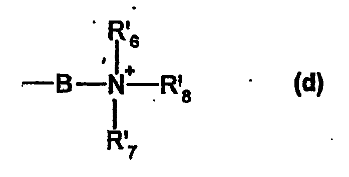
in der Formel:
- B bedeutet eine lineare oder verzweigte Alkylengruppe,
- R'₆ bedeutet Wasserstoff oder eine substituierte oder unsubstituierte Alkylgruppe,
- R'₇ und R'₈ bedeuten unabhängig voneinander eine substituierte oder unsubstituierte Alkylgruppe,
- R'₆ und R'₇ bilden gemeinsam mit dem Stickstoff einen 5-, 6-oder 7-gliedrigen Ring, der substituiert oder unsubstituiert ist und weitere Heteroatome enthalten kann, oder R'₆, R'₇ und R'₈ bilden gemeinsam einen Pyridiniumring,
- R'₃ bedeutet Wasserstoff, Halogen, (C₁₋₄)Alkyl, (C₁₋₄)Alkoxy,
- W ist eine Gruppe der folgenden Formel (e): 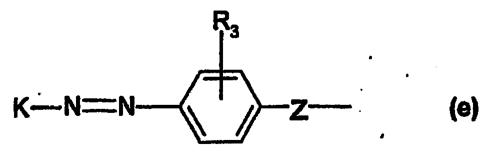 in der Formel:
- K ist ein Kupplungsrest,
- Z bedeutet eine Verknüpfungsgruppe, die unter den Gruppen der folgenden Formeln ausgewählt ist:
**-NR'**₉**-CO-;**
**-CO-NR'**₉**-NR'**₉**-CO-;** 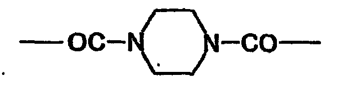 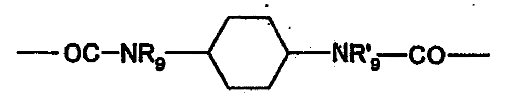 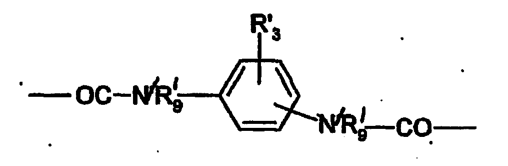 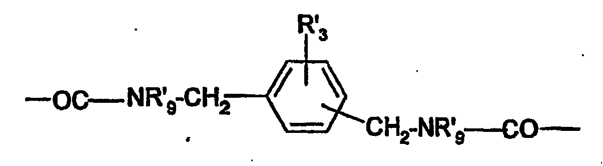
oder 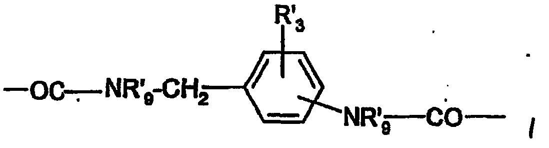 in denen R'₉ Wasserstoff, eine substituierte oder unsubstituierte (C₂₋₄)Alkylengruppe bedeutet, wobei die Alkylengruppe geradkettig oder verzweigt vorliegt und durch eine oder mehrere Gruppen unterbrochen sein kann, die ausgewählt sind unter -NR'9-, -O-, -S-.

2. Zusammensetzung nach Anspruch 1, **dadurch gekennzeichnet, dass** in der Formel (I)
- R₁ und R₂ bedeuten unabhängig voneinander Wasserstoff, eine mit Hydroxy oder (C₁₋₄)Alkoxy substituierte (C₁₋₄)Alkylgruppe und insbesondere Wasserstoff oder Methyl,
- Z ist eine lineare oder verzweigte oder cyclische Alkyl( C₂₋₈)-gruppe, die gegebenenfalls mit Hydroxy, Alkoxy, Halogen substituiert ist, wobei die Kette dieser Gruppe gegebenenfalls durch eine Gruppe -O- oder -NR₁- unterbrochen ist; eine 1,4-Phenylgruppe, eine 1,4-Naphthylgruppe, die gegebenenfalls mit Alkyl, Alkoxy, Halogen substituiert ist; wobei Z mit R₁, R₂ und den beiden Stickstoffatomen einen Piperazinring bilden kann, vorzugsweise:
- Z bedeutet eine unsubstituierte Phenylgruppe, eine Phenyl-oder Naphthylgruppe, die mit einer oder zwei Gruppen Methyl oder Methoxy substituiert ist, eine Piperazingruppe durch Verbindung mit R₁, R₂ und den beiden Stickstoffatomen, eine (C₂₋₄)Alkylengruppe, die unsubstituiert vorliegt oder mit einer oder zwei Hydroxygruppen substituiert ist,
- X ist eine Gruppe der Formel (b).

3. Zusammensetzung nach Anspruch 1, **dadurch gekennzeichnet, dass** in der Formel (II):
- Z1 bedeutet eine lineare oder verzweigte oder cyclische Alkyl(C₂₋₈)gruppe, die gegebenenfalls mit Hydroxy, Alkoxy, Halogen substituiert ist, wobei die Kette dieser Gruppe gegebenenfalls durch eine Gruppe -O- oder -NR₁- unterbrochen sein kann; einen Piperazinring, der mit R₁, R₂ und den beiden Stickstoffatomen gebildet wird, und vorzugsweise:
- Z₁ bedeutet eine (C₂₋₆)Alkylengruppe, die unsubstituiert vorliegt oder mit einer oder mehreren Hydroxygruppen substituiert ist, einen Piperazinring, der mit R₁, R₂ und den beiden Stickstoffatomen gebildet wird; und noch bevorzugter eine unsubstituierte (C₂₋₄)Alkylengruppe,
- R₃ und R₄ bedeuten Methyl oder Ethyl und R₅ und R₆ bedeuten Wasserstoff, Methyl oder Methoxy.

4. Zusammensetzung nach Anspruch 1, **dadurch gekennzeichnet, dass** in der Formel (III):
B bedeutet Ethylen, *n*-Propylen, Isopropylen oder *n*-Butylen,
K ist eine Kupplungsverbindung, die unter den Verbindungen der folgenden Formeln (f), (g) oder (h) ausgewählt ist: 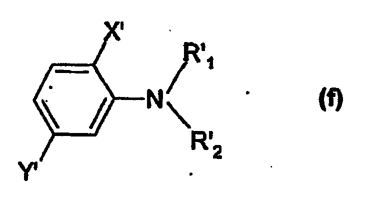 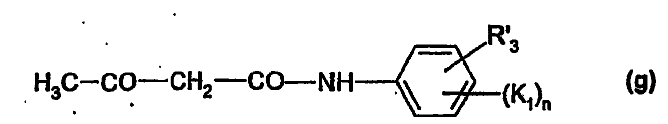 oder 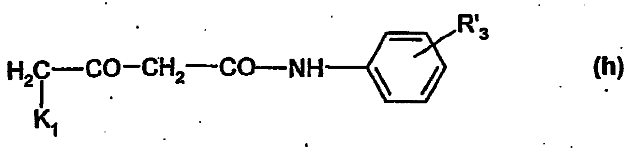
in den Formeln:
- X', Y' und R'₁ und R'₂ weisen die für die Formel (III) angegebenen Bedeutungen auf,
- n ist 1 oder 2,
- K₁ bedeutet die Gruppe der Formel:

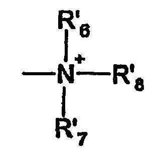

5. Zusammensetzung nach einem der Ansprüche 1 oder 2, **dadurch gekennzeichnet, dass** die Verbindung der Formel (I) oder der Formel (II) unter den Verbindungen der folgenden Formeln (I) 1 bis (I)7 ausgewählt ist:

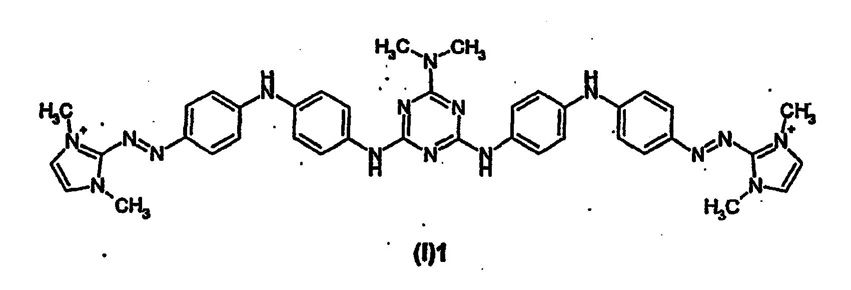

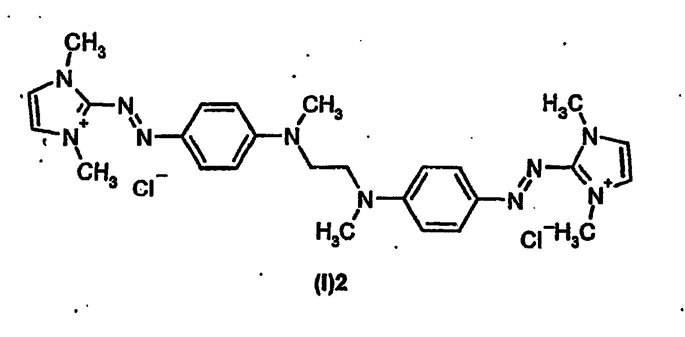

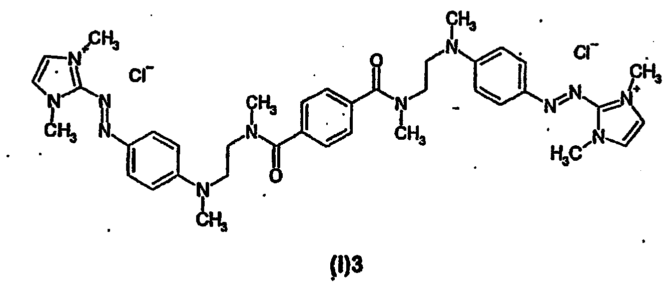

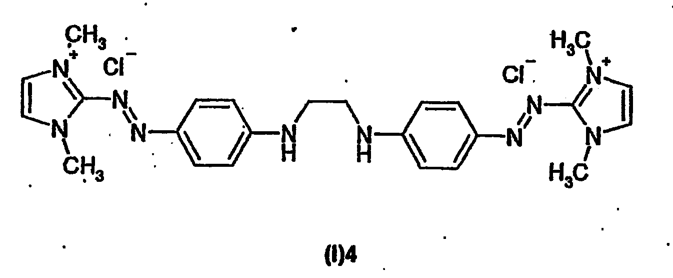

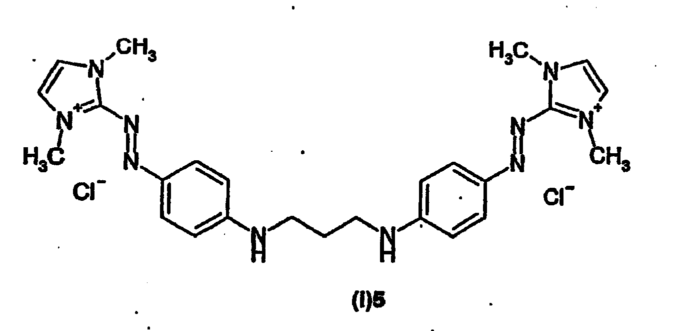

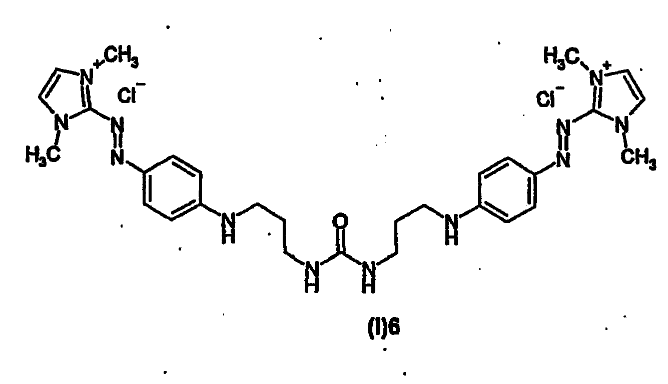

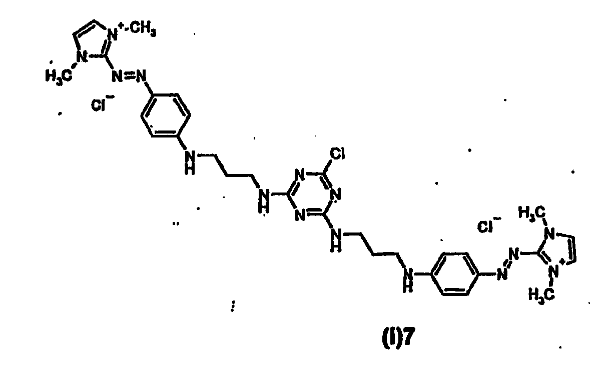

6. Zusammensetzung nach einem der Ansprüche 1 oder 4, **dadurch gekennzeichnet, dass** es sich bei der Verbindung der Formel (III) um die folgende Verbindung handelt:

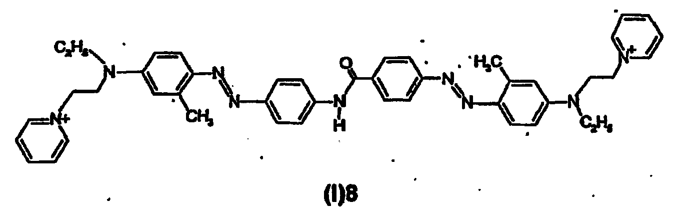

7. Zusammensetzung nach einem der vorhergehenden Ansprüche, **dadurch gekennzeichnet, dass** der oder die Direktfarbstoffe der Formeln (I), (II) oder (III) in einem Mengenanteil enthalten sind, der im Bereich von 0,01 bis 40 Gew.-%, bezogen auf das Gesamtgewicht der Zusammensetzung, liegt.

8. Zusammensetzung nach Anspruch 7, **dadurch gekennzeichnet, dass** der oder die Direktfarbstoffe der Formeln (I), (II) oder (III) in einer Menge enthalten sind, die im Bereich von 0,1 bis 20 Gew.-%, bezogen auf das Gesamtgewicht der Zusammensetzung, liegt.

9. Zusammensetzung nach einem der vorhergehenden Ansprüche, **dadurch gekennzeichnet, dass** der Oxidationsfarbstoff unter den Oxidationsbasen und/oder Kupplern ausgewählt ist.

10. Zusammensetzung nach Anspruch 9, **dadurch gekennzeichnet, dass** sie mindestens eine Oxidationsbase enthält.

11. Zusammensetzung nach den Ansprüchen 9 oder 10, **dadurch gekennzeichnet, dass** die Oxidationsbasen unter den ortho- oder para-Phenylendiaminen, Doppelbasen, ortho- oder para-Aminophenolen und heterocyclischen Basen sowie den Additionssalzen dieser Verbindungen mit einer Säure ausgewählt sind.

12. Zusammensetzung nach Anspruch 11, **dadurch gekennzeichnet, dass** die para-Phenylendiamine unter den Verbindungen der folgenden Struktur (V) ausgewählt sind: 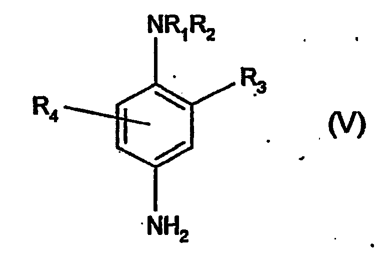 in der Formel:
R₁ bedeutet ein Wasserstoffatom, eine Alkyl(C₁₋₄)gruppe, eine Monohydroxyalkyl(C₁₋₄)gruppe, eine Polyhydroxyalkyl(C₂₋₄)gruppe, eine Alkoxy(C₁₋₄)alkyl(C₁₋₄)gruppe, eine mit einer Stickstoff enthaltenden Gruppe substituierte Alkyl(C₁₋₄)gruppe, Phenyl oder 4'-Aminophenyl;
R₂ bedeutet ein Wasserstoffatom, eine Alkyl(C₁₋₄)gruppe, eine Monohydroxyalkyl(C₁₋₄)gruppe oder eine Polyhydroxyalkyl(C₂₋₄)-gruppe, eine Alkoxy(C₁₋₄)alkyl(C₁₋₄)gruppe oder eine Alkyl(C₁₋₄)-gruppe, die mit einer Stickstoff enthaltenden Gruppe substituiert ist;
R₁ und R₂ können auch mit dem Stickstoffatom, von dem sie getragen werden, einen 5- oder 6-gliedrigen stickstoffhaltigen Heterocyclus bilden, der gegebenenfalls mit einer oder mehreren
Gruppen Alkyl, Hydroxy oder Ureido substituiert ist;
R₃ bedeutet ein Wasserstoffatom, ein Halogenatom wie Chlor, eine Alkyl(C₁₋₄)gruppe, Sulfo, Carboxy, Monohydroxyalkyl(C₁₋₄) oder Hydroxyalkoxy(C₁₋₄), Acetylaminoalkoxy(C₁₋₄), Mesylaminoalkoxy(C₁₋₄) oder Carbamoylaminoalkoxy(C₁₋₄),
R₄ ist ein Wasserstoffatom, ein Halogenatom oder eine Alkyl-(C₁₋₄)gruppe.

13. Zusammensetzung nach Anspruch 11, **dadurch gekennzeichnet, dass** die Doppelbasen unter den Verbindungen der folgenden Struktur (VI) ausgewählt sind: 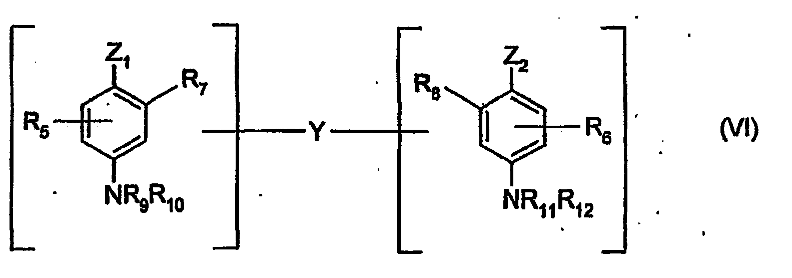 in der Formel:
- Z₁ und Z₂, die gleich oder verschieden sind, bedeuten eine Gruppe Hydroxy oder -NH₂, die mit einer C₁₋₄-Alkylgruppe oder einer Verbindungsgruppe Y substituiert sein können;
- die Verbindungsgruppe Y ist eine Alkylenkette mit 1 bis 14 Kohlenstoffatomen, die linear oder verzweigt ist und durch eine oder mehrere stickstoffhaltige Gruppen und/oder durch ein oder mehrere Heteroatome, wie Sauerstoff, Schwefel oder Stickstoff, unterbrochen oder abgeschlossen sein kann und gegebenenfalls mit einer oder mehreren Gruppen Hydroxy oder Alkoxy(C₁₋₆) substituiert ist;
- R₅ und R₆ bedeuten ein Wasserstoffatom, ein Halogenatom, eine Alkyl(C₁₋₄)gruppe, eine Monhydroxyalkyl(C₁₋₄)gruppe, eine Polyhydroxyalkyl(C₂₋₄)gruppe, eine Aminoalkyl(C₁₋₄)gruppe oder eine Verbindungsgruppe Y;
- R₇, R₈, R₉, R₁₀, R₁₁ und R₁₂, die gleich oder verschieden sind, bedeuten ein Wasserstoffatom, eine Verbindungsgruppe Y oder eine Alkyl(C₁₋₄)gruppe; mit der Maßgabe, dass die Verbindungen der Formel (VI) nur eine Verbindungsgruppe Y pro Molekül aufweisen.

14. Zusammensetzung nach Anspruch 11, **dadurch gekennzeichnet, dass** die para-Aminophenole unter den Verbindungen der folgenden Struktur (VII) ausgewählt sind: 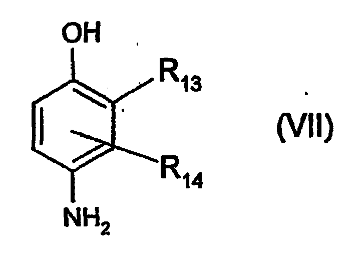 in der Formel:
R₁₃ bedeutet ein Wasserstoffatom, ein Halogenatom wie Fluor, eine Alkyl(C₁₋₄)gruppe, eine Monohydroxyalkyl(C₁₋₄)gruppe, eine Alkoxy(C₁₋₄)alkyl(C₁₋₄)gruppe oder eine Aminoalkyl(C₁₋₄)gruppe oder eine Hydroxyalkyl(C₁₋₄)aminoalkyl(C₁₋₄)gruppe,
R₁₄ ist ein Wasserstoffatom oder ein Halogenatom wie Fluor, eine Alkyl(C₁₋₄)gruppe, eine Monohydroxyalkyl(C₁₋₄)gruppe, eine Polyhydroxyalkyl(C₂₋₄)gruppe, eine Amino(C₁₋₄)gruppe, eine Cyanoalkyl(C₁₋₄)gruppe oder eine Alkoxy(C₁₋₄)alkyl(C₁₋₄)gruppe.

15. Zusammensetzung nach Anspruch 11, **dadurch gekennzeichnet, dass** die heterocyclischen Basen unter den Pyridinderivaten, Pyrimidinderivaten und darunter Pyrazolopyrimidinen und Pyrazolderivaten ausgewählt sind.

16. Zusammensetzung nach einem der Ansprüche 11 bis 15, **dadurch gekennzeichnet, dass** die Oxidationsbasen in Konzentrationen von 0,0005 bis 12 Gew.-%, bezogen auf das Gesamtgewicht der Zusammensetzung, vorliegen.

17. Zusammensetzung nach Anspruch 9, **dadurch gekennzeichnet, dass** die Kuppler unter den meta-Phenylendiaminen, meta-Aminophenolen, meta-Dihydroxybenzolen, heterocyclischen Kupplern und den Additionssalzen dieser Verbindungen mit einer Säure ausgewählt sind.

18. Zusammensetzung nach einem der Ansprüche 9 oder 17, **dadurch gekennzeichnet, dass** die Kuppler in Konzentrationen von 0,0001 bis 10 Gew.-%, bezogen auf das Gesamtgewicht der Zusammensetzung, vorliegen.

19. Zusammensetzung nach einem der Ansprüche 11 bis 17, **dadurch gekennzeichnet, dass** die Additionssalze der Oxidationsfarbstoffe mit einer Säure unter den Hydrochloriden, Hydrobromiden, Sulfaten, Tartraten, Lactaten und Acetaten ausgewählt sind.

20. Zusammensetzung nach einem der vorhergehenden Ansprüche, **dadurch gekennzeichnet, dass** sie ferner in Mengenanteilen von 0,01 bis 10 Gew.-%, bezogen auf das Gesamtgewicht der Zusammensetzung, mindestens ein kationisch oder amphoteres Polymer enthält, das ausgewählt ist unter:
-1 / Homopolymeren von Dimethyldiallylammoniumchlorid;
-2/ Polymeren mit Wiederholungseinheiten der folgenden Formeln (W) oder (U): 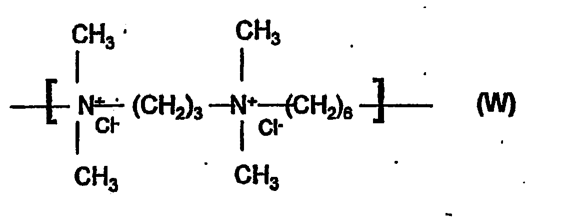 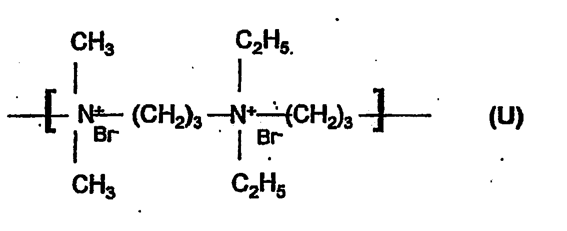
-3/ Polymeren mit Einheiten der folgenden Formel (IX): 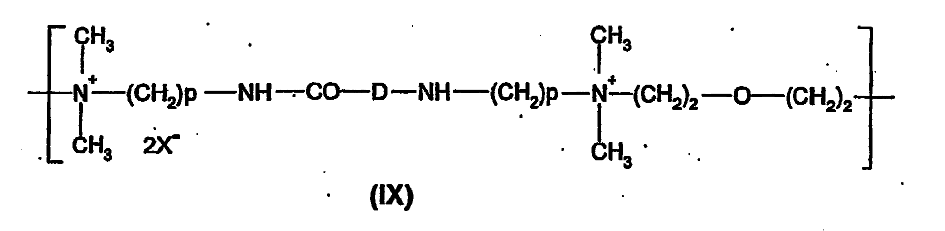 bei der p 3 ist und
a) D nicht vorhanden ist, X ein Chloratom bedeutet,
b) D eine Gruppe -(CH₂)₄-CO- bedeutet, X ein Chloratom ist,
c) D eine Gruppe -(CH₂)₇-CO- bedeutet, X ein Chloratom ist,
d) ein "Blockcopolymer", das aus Einheiten gebildet ist, die den unter den Punkten a) und b) beschriebenen Polymeren entsprechen;
-4/ Copolymeren von Acrylsäure und Dimethyldiallylammoniumchlorid.

21. Zusammensetzung nach einem der vorhergehenden Ansprüche, **dadurch gekennzeichnet, dass** sie mindestens einen grenzflächenaktiven Stoff enthält, der unter den anionischen grenzflächenaktiven Stoffen, kationischen grenzflächenaktiven Stoffen, nichtionischen grenzflächenaktiven Stoffen oder amphoteren grenzflächenaktiven Stoffen ausgewählt ist.

22. Zusammensetzung nach einem der vorhergehenden Ansprüche, **dadurch gekennzeichnet, dass** sie ferner mindestens ein Reduktionsmittel in Mengenanteilen von 0,5 bis 3 Gew.-%, bezogen auf das Gesamtgewicht der Zusammensetzung, enthält.

23. Verfahren zum oxidativen Färben von menschlichen Keratinfasern und insbesondere Haaren, das darin besteht, auf die trockenen oder feuchten Haare bei Umgebungstemperatur eine gebrauchsfertige Farbmittelzusammensetzung aufzutragen, die bei der Anwendung durch bedarfsgemäßes Mischen einer Farbmittelzusammensetzung, die in einem zum Färben geeigneten Medium mindestens einen Oxidationsfarbstoff und mindestens eine dikationische Verbindung der Formel (I), (II) oder (III) enthält, wie sie in einem der Ansprüche 1 bis 6 definiert sind, und einer oxidierenden Zusammensetzung gebildet wird, und während einer Einwirkzeit von 5 bis 60 Minuten einwirken zu lassen, die Fasern zu spülen, anschließend gegebenenfalls mit Haarwaschmittel zu waschen, nochmals zu spülen und die Fasern zu trocknen.

24. Verfahren nach Anspruch 23, **dadurch gekennzeichnet, dass** die Einwirkzeit im Bereich von 10 Sekunden bis 5 Minuten und insbesondere 30 Sekunden bis 2 Minuten liegt.

25. Vorrichtung zum oxidativen Färben von menschlichen Keratinfasern und insbesondere Haaren, die eine erste Abteilung aufweist, die in einem zum Färben geeigneten Medium mindestens einen Oxidationsfarbstoff und mindestens eine dikationische Verbindung der Formeln (I), (II) oder (III) enthält, wie sie in einem der Ansprüche 1 bis 6 definiert sind, und eine zweite Abteilung, die ein Oxidationsmittel enthält.
